# FASCICULE DE BREVET EUROPEEN

(11) **EP 0 638 568 B1**
(45) Date de publication et mention de la délivrance du brevet: **09.10.1996**
(21) Numéro de dépôt: 94401602.1
(22) Date de dépôt: 12.07.1994
(51) Int. Cl.: C07D 403/04, C07D 403/06, C07D 413/04, C07D 413/06, A61K 31/495

(54) **Pipérazines substituées, leur procédé de préparation et les compositions pharmaceutiques les contenant**
Substituierte Piperazine, ihre Verfahrensherstellung und die enthaltenen pharmazeutischen Zusammenfassungen
Substituted piperazines, their process of preparation and the pharmaceutical compositions containing them

(30) Priorité: 23.07.1993 FR 9309088
(43) Date de publication de la demande: 15.02.1995
(73) Titulaire: ADIR ET COMPAGNIE, F-92415 Courbevoie Cédex (FR)
(72) Inventeur: Godfroid, Jean-Jacques, F-75020 Paris (FR); Lamouri, Aazdine, F-92220 Bagneux (FR); Touboul, Estera, F-75020 Paris (FR); Wang, Xuan, FR-L'Hay les Roses (FR); Renard, Pierre, F-78000 Versailles (FR); Pfeiffer, Bruno, F-95600 Eaubonne (FR); Guardiola, Béatrice, F-92210 Saint Cloud (FR)

(56) Documents cités:
- EP-A- 0 324 520
- CHEMICAL ABSTRACTS, vol. 108, no. 3, 18 Janvier 1988, Columbus, Ohio, US; abstract no. 21816j,
- JOURNAL OF MEDICINAL CHEMISTRY, vol.33, no.10, 1990, WASHINGTON US pages 2690 - 2697 L.J. STREET ET AL.

## Description

La présente invention concerne de nouvelles pipérazines substituées, leur procédé de préparation et les compositions pharmaceutiques qui les contiennent.

La littérature fournit quelques exemples de pipérazines substituées. On pourra notamment citer les travaux de L. J. Street et al. (J. Med. Chem., 33(10), (1990), 2690-2697) dans lesquels sont présentés des pipérazines, substituées en position 2 par des groupements oxadiazoles, et utiles en tant qu'agonistes des récepteurs muscariniques centraux.

D'autres publications décrivent des dérivés de pipérazines utiles dans le traitement de la maladie de Parkinson (Indian J. Chem., 28B(9), (1989), 745-750) ou bien possédant une activité hypotensive (Sci. Pharm., 53(3), (1985), 133-138).

La demanderesse a découvert une nouvelle série de pipérazines substituées douées de propriétés pharmacologiques, inattendues pour ce type de molécules, et très intéressantes pour le traitement du diabète non insulino-dépendant.

Plus précisément l'invention concerne de nouvelles pipérazines de formule générale (I) : 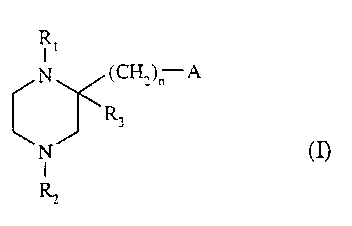 dans laquelle :
- R₁ et R₂ indépendamment l'un de l'autre sont choisis parmi l'hydrogène, les radicaux alkyles, cycloalkyles, monocycloalkylalkyles, dicycloalkylalkyles, aryles et arylalkyles, chacun de ces radicaux étant éventuellement substitué,
- R₃ est choisi parmi l'hydrogène et un radical alkyle,
- n prend indifféremment une valeur choisie parmi 0, 1 et 2,
- A représente le radical 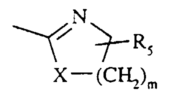 dans lequel
   - X représente l'oxygène ou le groupement N-R₄,
   - m prend une valeur choisie parmi 1 et 2,
   - R₄ est choisi parmi l'hydrogène et les radicaux alkyles, alkoxycarbonyles et aryloxycarbonyles éventuellement substitués, et
   - R₅ est choisi parmi l'hydrogène et un radical alkyle,
   étant entendu que :
- les termes "alkyle", "alkoxy", "monocycloalkylalkyle", "dicycloalkylalkyle", "arylalkyle" et "alkoxycarbonyle" désignent des radicaux dont la chaîne hydrocarbonée comporte de 1 à 10 atomes de carbone en chaîne droite ou ramifiée,
- le terme "cycloalkyle" désigne des radicaux hydrocarbonés cycliques comportant de 3 à 8 atomes de carbone,
- les termes "aryle", "arylalkyle" et "aryloxycarbonyle" concernent des radicaux dont la partie aromatique est un radical phényle ou un radical naphtyle,
- l'expression "éventuellement substitué" signifie que les radicaux ainsi qualifiés peuvent être substitués par une ou plusieurs entités chimiques choisies parmi les radicaux alkyles, hydroxy, alkoxy, halogéno, halogénoalkyles, polyhalogénoalkyles, nitro, amino, alkylamino et polyalkylamino,
leurs stéréoisomères, N-oxydes et sels d'addition à un acide ou à une base, pharmaceutiquement acceptables.

La présente invention s'étend également au procédé de préparation des composés de formule (I), caractérisé en ce que :
a) - soit, le composé de formule (II) :

   C₆H₅-CH₂-NH-CH₂-CH₂-NH-CH₂-C₆H₆ (II)

   est porté à reflux dans un solvant aromatique, tel que le benzène ou le toluène, en présence de triéthylamine avec le dibromure de formule (III) : 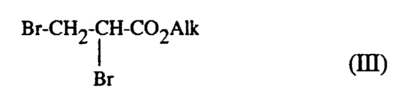 dans laquelle Alk représente un radical alkyle linéaire ou ramifié comportant de 1 à 4 atomes de carbone,
   pour conduire au composé de formule (IV) : 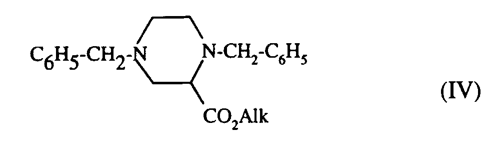 dans laquelle Alk est tel que défini précédemment,
   qui est ensuite débenzylé, par action d'hydrogène en présence de catalyseur, tel que le palladium sur charbon dans l'éthanol absolu, pour conduire au composé de formule (V) : 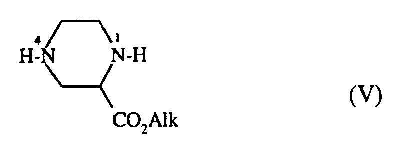 dans laquelle Alk est tel que défini précédemment,
   qui est transformé ensuite en composé de formule (Va), (Vb) ou (Vc) : 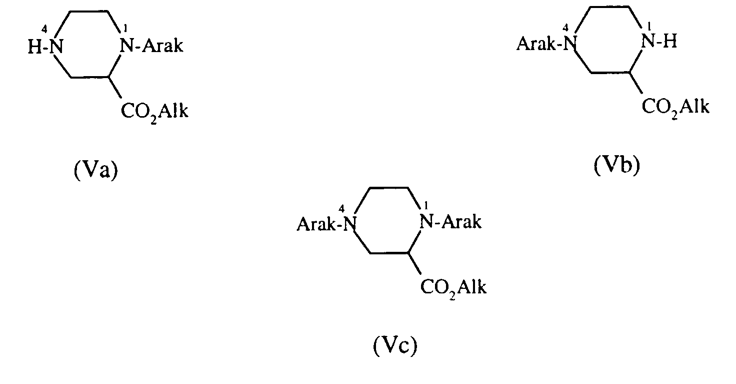 où Arak représente un radical arylalkyle éventuellement substitué comme défini précédemment, et Alk est tel que défini précédemment,
   - pour le composé de formule (Va) :
      après protection de l'atome d'azote 4 par le chlorure de triphénylméthyle en solvant approprié tel que le chlorure de méthylène, en présence de triéthylamine, par traitement par Arak-Cl en présence de carbonate de potassium et d'une quantité catalytique d'iodure de potassium, en solvant approprié, tel que l'acétone à froid, puis déprotection de l'atome d'azote 4, par le chlorure d'hydrogène gazeux en solvant adéquat, tel que l'éthanol ou un mélange acide chlorhydrique concentré/acétone,
   - pour les composés de formules (Vb) et (Vc) :
      par traitement du composé de formule (V) directement par Arak-Cl dans le toluène à reflux en présence de triéthylamine, puis séparation par chromatographie sur colonne de gel de silice des dérivés mono- et disubstitués,
b) - soit, le composé de formule (VI) : 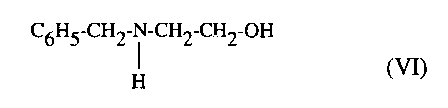 est éventuellement traité par une cétone ou un aldéhyde, en milieu acide, par exemple l'acide acétique ou formique, et alcoolique, par exemple le méthanol, la cétone ou l'aldéhyde choisi étant l'homologue du radical R'₁ (R'₁ ayant la même définition que R₁, l'hydrogène excepté) que l'on souhaite greffer sur l'atome d'azote, par exemple le formaldéhyde lorsque R'₁ représente le radical méthyle, la cyclohexanone lorsque R'₁ représente le radical cyclohexyle, afin d'obtenir le composé de formule (VII) : 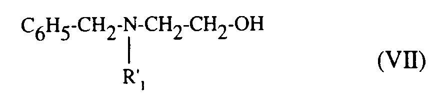 dans laquelle R'₁ est tel que défini précédemment,
   composés de formules (VI) et (VII), qui, traités par un agent de chloration, comme le chlorure de thionyle, fournissent les dérivés chlorés correspondants, puis sous action d'une solution aqueuse saturée d'ammoniaque conduisent au composé de formule (VIII) : 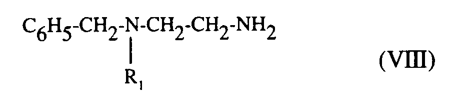 dans laquelle R₁ est tel que défini précédemment,
   pour être éventuellement traité ensuite, comme le composé de formule (VI) par l'homologue du radical R'₂, (R'₂ possédant la même définition que R₂, I'hydrogène excepté), sous forme d'aldéhyde ou de cétone, et conduire, après hydrogénation de la double liaison formée, par exemple par le borohydrure de sodium, à la diamine de formule (IX) : 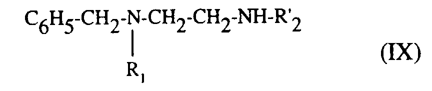 dans laquelle R₁ et R'₂ sont tels que définis précédemment,
   les composés de formules (VIII) et (IX) étant alors débenzylés selon le procédé décrit pour le composé de formule (IV) puis traités par le dibromure de formule (III) décrit précédemment pour donner après séparation des éventuels isomères par chromatographie et/ou cristallisation, le composé de formule (X) : 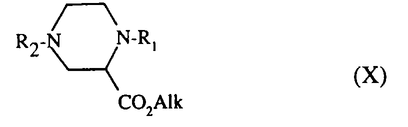 dans laquelle R₁, R₂ et Alk sont tels que définis précédemment,
c) - soit, le composé de formule (VI) : 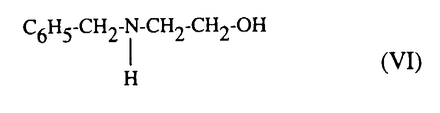 est éventuellement traité par une cétone ou un aldéhyde, en milieu acide, par exemple l'acide acétique, et alcoolique par exemple le méthanol, la cétone ou l'aldéhyde choisi étant l'homologue du radical R'₂ (R'₂ ayant la même définition que R₂, l'hydrogène excepté) que l'on souhaite greffer sur l'atome d'azote, par exemple le formaldéhyde lorsque R'₂ représente le radical méthyle, la cyclohexanone lorsque R'₂ représente le radical cyclohexyle, afin d'obtenir le composé de formule (XI) : 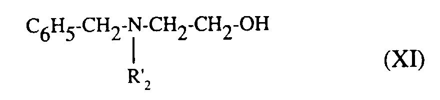 dans laquelle R'₂ est tel que défini précédemment,
   composés de formules (VI) et (XI) qui traités par un agent de chloration, comme le chlorure de thionyle, fournissent les dérivés chlorés correspondants qui, traités par la benzylamine conduisent à la diamine de formule (XII) : 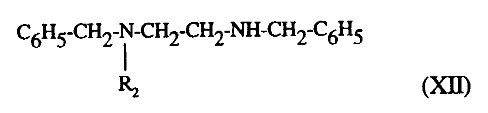 dans laquelle R₂ est tel que défini précémment,
   qui par réaction avec un dérivé halogéné de formule (XIII) : 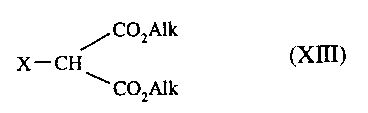 dans laquelle X représente un atome d'halogène et Alk est tel que défini précédemment,
   conduit à la diamine de formule (XIV) : 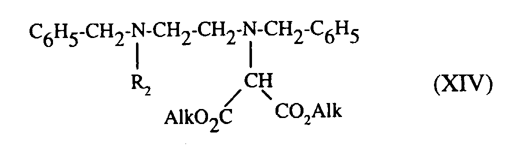 dans laquelle Alk et R₂ sont tels que définis précédemment,
   qui après métallation et réaction avec un dérivé halogéné de formule (XV) :

   X-R₃ (XV)

   dans laquelle X et R₃ sont tels que définis précédemment,
   conduit à la diamine de formule (XVI) : 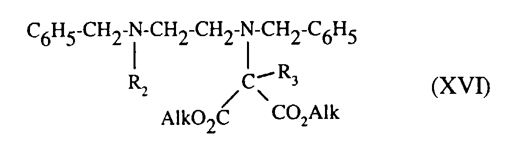 dans laquelle R₂, R₃ et Alk sont tels que définis précédemment,
   qui, après débenzylation catalytique et cyclisation, conduit à la pipérazinone de formule (XVII) : 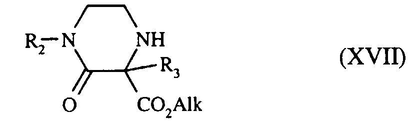 dans laquelle R₂, R₃ et Alk sont tels que définis précédemment,
   qui :
   - soit par action d'aldéhyde ou de cétone précurseurs du radical R'₁ selon la méthode décrite précédemment,
   - soit par alkylation directe avec un dérivé halogéné de formule (XVIII) :

      R'₁-X (XVIII)

      dans laquelle R'₁ et X sont tels que définis précédemment,
   conduit la pipérazinone de formule générale (XIX) : 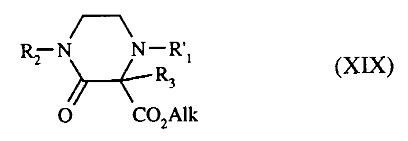 dans laquelle R'₁, R₂, R₃ et Alk sont tels que définis précédemment,
   pipérazinones de formules (XIX) et (XVII) qui, par réduction avec des hydrures de bore, conduisent à la pipérazine de formule (XX) : 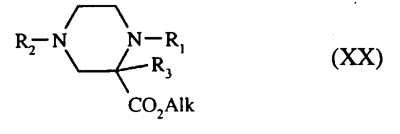 dans laquelle R₁, R₂, R₃ et Alk sont tels que définis précédemment,
   les amines secondaires des composés de formules (V), (Va) et (Vb) pouvant également être substituées :
   - soit par action d'aldéhydes ou de cétones précurseurs des radicaux R'₁ ou R'₂ selon la méthode décrite précédemment,
   - soit par alkylation directe avec un dérivé halogéné de formule (XXI) :

      R-X (XX)

      dans laquelle R a la même définition que celle de R'₁ ou R'₂ et X représente un halogène,
      avec, dans le cas des composés de formule (V), protection éventuelle au préalable de l'atome d'azote le plus réactif par un groupement triphénylméthyle, comme indiqué pour l'obtention du composé de formule (Va),
   les composés de formules (V), (Va), (Vb), (Vc), leurs éventuels produits de substitutions tels qu'envisagés précedemment, ainsi que les composés de formule (X) faisant également partie des composés de formule (XX),
   composés de formules (XX) qui peuvent éventuellement être transformés en leurs homologues supérieurs de formule (XXII) : 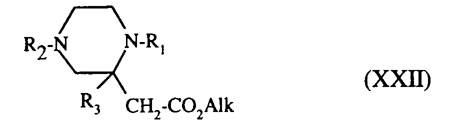 dans laquelle R₁, R₂, R₃ et Alk sont tels que définis précédemment,
   par traitement par un agent de réduction, par exemple l'hydrure double de lithium et d'aluminium, afin de transformer la fonction ester en fonction alcool, puis chloration, par exemple sous l'action de chlorure de thionyle, suivie d'un traitement par un cyanure d'alcalino-terreux, par exemple le cyanure de potassium, et finalement transformation du nitrile ainsi obtenu en ester sous l'action d'un alcool en milieu acide, par exemple, un mélange éthanol acide sulfurique concentré,
   composé de formule (XXII), qui, éventuellement soumis au même traitement, conduit à l'homologue de formule (XXIII) : 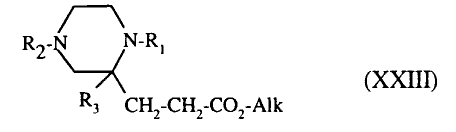 dans laquelle R₁, R₂, R₃ et Alk sont tels que définis précédemment,
   l'ensemble des composés de formule (XX), (XXII) et (XXIII) formant l'ensemble des composés de formule (XXIV) : 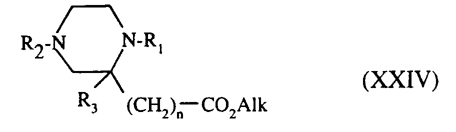 dans laquelle R₁, R₂, R₃, Alk et n sont tels que définis précédemment,
   qui sont soumis :
   **soit** : a) à l'action d'une diamine de formule (XXV) : 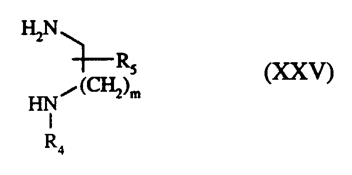 dans laquelle R₄, R₅ et m sont tels que définis précédemment,
      en présence d'un trialkylaluminium, par exemple le triméthylaluminium, en solvant aprotique anhydre, tel que le toluène, à reflux, pour conduire au composé de formule (Ia) : 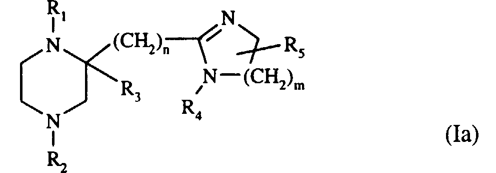 dans laquelle R₁, R₂, R₃, R₄, R₅, n et m sont tels que définis précédemment,
   **soit** : b) à l'action du composé de formule (XXVI) : 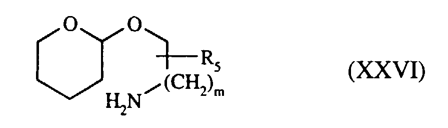 dans laquelle R₅ et m sont tels que définis précédemment,
   composé obtenu à partir de l'α-ω-hydroxy-amine correspondante, dont la fonction amine a été préalablement protégée en N,N-dibenzyl-α-ω-hydroxy-amine, sous l'action du chlorure de benzyle, puis protection de la fonction alcool par le dihydropyrane, puis hydrogénolyse en présence de palladium sur charbon afin de libérer la fonction amine,
   composé de formule (XXVI) réagissant sur le composé de formule (XXIV) dans les mêmes conditions que le composé de formule (XXV) pour conduire au composé de formule (XXVII) : 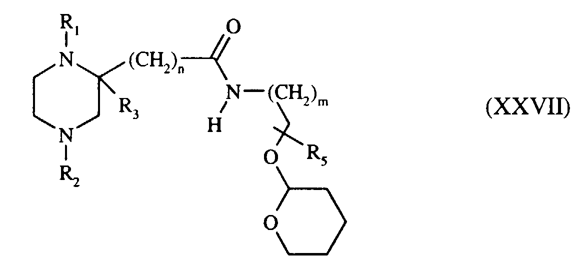 dans laquelle R₁, R₂, R₃, R₅, n et m sont tels que définis précédemment,
   composé, dont la fonction alcool est libérée en milieu acide alcoolique, par exemple un mélange acide chlorhydrique/méthanol, puis transformée en chlorure correspondant sous l'action de chlorure de thionyle pour permettre la cyclisation souhaitée, en milieu basique, hydroxyde de sodium par exemple, et obtenir le composé de formule (Ib) : 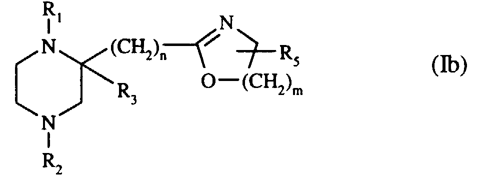 dans laquelle R₁, R₂, R₃, R₅, n et m sont tels que définis précédemment,
   l'ensemble des composés de formules (Ia) et (Ib) formant l'ensemble des composés de formule (I), qui sont purifiés et éventuellement séparés en leurs stéréoisomères par une technique classique de séparation et qui sont, si on le souhaite, transformés en leurs N-oxydes ou en leurs sels d'addition à un acide ou à une base, pharmaceutiquement acceptables.

Les composés de la présente invention sont doués d'une activité anti-diabétique très puissante. Ils améliorent très fortement la tolérance au glucose in vivo chez des modèles animaux de diabète de type II (non-insulino-dépendants) que ce soit par voie orale, intra-péritonéale ou intra-veineuse.

Les composés de l'invention ne semblent pas posséder d'affinité importante pour les récepteurs α (alpha) et β (bêta) adrénergiques.
Après avoir été testés par voie orale chez le rat, les composés de l'invention se sont révélés non toxiques jusqu'à une dose de 1 g/kg. Leur forte activité associée à leur faible toxicité rend les composés de l'invention particulièrement intéressants pour le traitement du diabète non insulino-dépendant.

La présente invention a également pour objet les compositions pharmaceutiques contenant au moins un composé de formule (I), ou un de ses N-oxydes ou sels d'addition à un acide ou à une base pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiées, les gélules, les suppositoires, les capsules, les crèmes, pommades, gels dermiques,...

La posologie utile varie selon l'âge et le poids du patient, la voie d'administration, la nature de l'affection et des traitements éventuels associés et s'échelonne entre 0,5 mg et 1 gramme par 24 heures.

Les exemples suivants illustrent l'invention sans toutefois la limiter en aucune façon.

Les matières premières sont disponibles ou préparées à partir de modes opératoires connus.

### Préparation A :

### 1,4-Dibenzyl-2-éthoxycarbonylpipérazine (dichlorhydrate)

A une solution chaude de 72 g (0,3 mole) de N,N'-dibenzyléthylènediamine, 700 ml de benzène et 50 ml (0,36 mole) de triéthylamine, sont ajoutés goutte à goutte, mais assez rapidement, 80 g (0,31 mole) de 2,3-dibromo- propionate d'éthyle dilués dans 300 ml de benzène. Après addition, le reflux est maintenu pendant 3 heures (le bromhydrate de la triéthylamine précipite dans le mélange réactionnel). Le mélange est refroidi, filtré sur verre frité et le solide lavé à l'éther. Le filtrat est lavé avec 200 ml de solution saturée d'hydrogénocarbonate de sodium puis 2 fois avec 100 ml d'eau. Les phases organiques sont séchées sur sulfate de magnésium et les solvants évaporés sous vide. Environ 100 ml de toluène sont ajoutés et on continue l'évaporation. Le résidu obtenu est dilué dans 400 ml d'éthanol absolu et saturé de chlorure d'hydrogène gazeux. Le sel précipite par addition d'éther. Après filtration, lavage à l'éther et séchage, on obtient 100 à 110 g de cristaux blancs.
Rendement : 89 %

### Préparation B :

### 2-Ethoxycarbonylpipérazine (dichlorhydrate)

A 41,1 g (0,1 mole) du composé obtenu à la préparation A mis en suspension dans 300 ml d'éthanol absolu sont ajoutés 600 mg de palladium sur charbon à 10 %. Le mélange est agité et chauffé à 40°C pendant 12 heures sous atmosphère d'hydrogène.Le catalyseur est éliminé par filtration et le composé du titre précipite par addition de l'éther. On obtient 20 à 21 g de cristaux blancs.
Rendement : 86-91 %

### Préparation C :

### 2,4-Diméthyl-2-éthoxycarbonyl- 1 -(2,4-dichlorobenzyl)pipérazine

### - Etape a : N-B N-Benzyl-N-méthylaminoéthanol

Un mélange constitué de 151 g (1 mole) de N-benzylaminoéthanol, 120 ml d'acide formique à 90 %, 120 ml d'une solution aqueuse de formaldéhyde à 35 % et de 11 méthanol est chauffé à reflux pendant 20 heures. Le solvant est évaporé sous vide et le résidu est traité par une solution aqueuse d'hydroxyde de sodium puis extrait au chlorure de méthylène. Après traitement habituel, le produit est purifié par distillation. On récupère 138 g de produit attendu.
Point d'ébullition : (2021Pa): 140-142°C
Rendement : 84 %

### - Etape b : N-Benzyl-N-(2-chloro)éthylméthylamine

A 130 g (0,79 mole) de composé obtenu à l'étape a, dissous dans 400 ml de trichlorométhane sont ajoutés goutte à goutte 70 ml (0,96 mole) de chlorure de thionyle dans 70 ml de trichlorométhane. Le mélange est agité 15 h. à température ambiante et le solvant évaporé sous vide. Le résidu est recristallisé dans un mélange acétone-méthanol. On obtient 155 g de critaux blancs correspondant au produit attendu.
Point de fusion : 140,5°C

### Etape c : N,N'-Dibenzyl-N-méthyl-1,2-diaminoéthane

A 150 g (1,4 mole) de benzylamine diluée dans 200 ml d'éthanol chaud sont ajoutés goutte à goutte 128 g (0,7 mole) de composé obtenu à l'étape b dissous dans 600 ml d'éthanol. Le mélange est agité à température ambiante pendant 24 heures et le solvant évaporé sous vide. Le chlorhydrate de benzylamine cristallise dans un mélange de dichlorométhane et d'acétone, et est éliminé par filtration. Les filtrats sont saturés par chlorure d'hydrogène gazeux et le chlorhydrate du composé attendu cristallise dans un mélange dichlorométhane/éther. On récupère 105 g de cristaux blancs.
Rendement : 46 %

### Etape d : 2-{N-[2-N'-méthylbenzylamino)éthyl]benzylamino}malonate de diéthyle

100 g (0,5 mole) de composé obtenu à l'étape c dissous dans 600 ml de dichlorométhane sont ajoutés à 62 g (0,3 mole) de bromomalonate d'éthyle. La réaction est agitée à température ambiante pendant 24 heures et la phase organique est lavée à l'eau. Le traitement habituel de la phase organique fournit 80 g du composé attendu sous forme d'une huile incolore.
Rendement : 65 %

### - Etape e : 2-{N-[2-(N'-méthylbenzylamino)éthyl]benzylamino}-2-méthylmalonate de diéthyle

7,8 g d'hydrure de sodium à 60 % dans l'huile et 70 ml de diméthylformamide sont ajoutés, goutte à goutte et à froid, à 80 g (0,195 mole) du diester obtenu précédemment dilué dans 140 ml de diméthylformamide. Lorsque le dégagement gazeux est terminé, 12,14 ml (0,195 mole) d'iodure de méthyle dans 35 ml de diméthylformamide sont ajoutés goutte à goutte. La réaction est agitée à température ambiante pendant une nuit. Le précipité formé est filtré après addition d'éther. Le traitement habituel de la phase organique conduit à 75 g du composé attendu.
Rendement : 90 %

### - Etape f : 1,3-Diméthyl-2-oxopipérazine-3-carboxylate d'éthyle

75 g de composé obtenu à l'étape e dans 500 ml d'éthanol absolu et 100 mg de palladium sur charbon à 10 % sont agités et chauffés à 45°C sous atmosphère d'hydrogène pendant 48 h. Après filtration le solvant est évaporé et le résidu est purifié par chromatographie sur colonne de gel de silice. (éluant dichlorométhane puis méthanol 5 %). 20 g d'huile incolore sont obtenus.
Rendement : 60 %

### - Etape g : 1-(2,4-Dichlorobenzyl)-2,4-diméthyl-3-oxopiperazine-2-carboxylate d'éthyle

A 20 g (0,107 mole) de composé obtenu à l'étape précédente solubilisés dans 650 ml d'acétone sont ajoutés 43 g de carbonate de potassium et 4,3 g d'iodure de potassium. Une solution de 0,128 mole de chlorure de 2,4-dichlorobenzyle dans 100 ml d'acétone, est ajoutée goutte à goutte. Le mélange est alors agité à température ambiante pendant plusieurs heures. Les sels sont éliminés par filtration. Le traitement habituel de la phase organique fournit le composé attendu qui cristallise dans un mélange éther/hexane.

### - Etape h : 1-(2,4-Dichlorobenzyl)-2,4-diméthylpipérazine-2-carboxylate d'éthyle

A 1,1 g de borohydrure de sodium sec, mis en suspension dans 35 ml de tétrahydrofurane fraîchement distillé, sont ajoutés goutte à goutte et à froid, 4,4 ml de trifluorure de bore fraîchement distillé. La réaction est agitée à 0°C pendant 2 heures puis 3,5 g (0,01 mole) du composé obtenu à l'étape g dans 100 ml de tétrahydrofurane sont additionés. Le mélange est alors chauffé à reflux pendant une heure puis 50 ml d'une solution 2N d'acide chlorhydrique sont ajoutés à froid. Le milieu est neutralisé par de l'hydroxyde de soidum puis extrait à l'éther. Le traitement habituel de la phase organique fournit un produit brut qui est purifié sur colonne de gel de silice (éluant : trichlorométhane). 1 g de produit attendu, pur, sous forme d'huile incolore, est obtenu.
Rendement : 30 %

### EXEMPLE 1 :

### 1-(2-Chlorobenzyl)-4-méthyl-2-(4,5-dihydro[1H]imidazol-2-yl)pipérazine

### - Etape a : 4-Triphénylméthyl-2-éthoxycarbonylpipérazine

A un mélange de 23,1 g (0,1 mole) du composé obtenu à la préparation B, 400 ml de dichlorométhane et 55 ml (0,4 mole) de triéthylamine, refroidi à -10°C, sont ajoutés, goutte à goutte, 28 g (0,1 mole) de chlorure de triphénylméthane dilués dans 300 ml de dichlorométhane. Après addition, l'agitation est maintenue durant une nuit. 21 g de carbonate de sodium dissous dans 200 ml d'eau sont alors ajoutés. La phase organique est décantée, lavée avec une solution saturée de chlorure de sodium, séchée sur sulfate de magnésium et le solvant évaporé sous vide. On ajoute environ 50 ml de toluène et l'on continue l'évaporation. Le résidu cristallise dans l'hexane-éther (9:1) et fournit 26 g de cristaux blancs. Le filtrat est purifié par chromatographie sur colonne de gel de silice, éluant éther de pétrole/éther (7:3) d'abord puis éther pur. 6 g de produit cristallisé pur sont obtenus.
Rendement : 80 %

### - Etape b : 1-(2-Chlorobenzyl)-2-éthoxycarbonylpipérazine

Un mélange de 40 g (0,1 mole) de composé obtenu à l'étape a, 400 ml d'acétone, 40 g de carbonate de potassium, 4 g d'iodure de potassium et de 19 g (0,12 mole) de chlorure de 2-chlorobenzyle est agité 15 heures à température ambiante. Après traitement habituel de la phase organique, les solvants sont évaporés sous vide, le résidu obtenu est dissous dans 300 ml d'acétone et 300 ml d'acétone contenant 25 ml d'acide chlorhydrique concentré sont ajoutés. Le mélange est agité 2 heures à température ambiante. Le traitement de la phase organique fournit 19 g du produit attendu.
Rendement : 80 %

### - Etape c : 1-(2-Chlorobenzyl)-4-méthyl-2-éthoxycarbonylpipérazine

Un mélange de 28 g (0,1 mole) du composé obtenu à l'étape b, 120 ml de méthanol, 12 ml de formaldéhyde à 37 % et 12 ml d'acide formique, est chauffé 20 heures à reflux. Le solvant est évaporé sous vide, le résidu obtenu est repris par l'éther et une solution saturée d'hydrogénocarbonate de sodium. La phase organique est lavée, neutralisée puis extraite à l'éther. Après séchage sur sulfate de magnésium et évaporation du solvant, le résidu est dissous dans 200 ml d'éthanol absolu et saturé d'acide chlorhydrique gazeux. Le dichlorhydrate précipite par addition d'éther. 32 g de cristaux blancs sont isolés.
Rendement : 86,5 %

### - Etape d : 1-(2-Chlorobenzyl)-4-méthyl-2-(4,5-dihydro[1H]imidazol-2-yl)pipérazine

A 200 ml de toluène anhydre sont ajoutés 75 ml de solution 2M de triméthylaluminium dans le toluène. Le mélange est refroidi dans la glace et 4 ml (0,06 mole) d'éthylène diamine sont ajoutés. Le mélange est ramené à température ambiante, puis 9,5 g (0,032 mole) de composé obtenu à l'étape précédente sont ajoutés, dans 80 ml de toluène. L'ensemble est chauffé à reflux pendant 5 heures. Toutes les manipulations sont effectuées sous atmosphère d'argon. Après une nuit à température ambiante, le mélange réactionnel est hydrolysé avec 100 ml de solution méthanol-eau (8:2). Après filtration, on évapore les solvants sous vide, le résidu est dissous dans le dichlorométhane et la phase organique est lavée avec une solution saturée de chlorure de sodium, puis avec une solution d'acide chlorhydrique 2N. L'extraction au dichlorométhane fournit 7 g du produit titre qui est purifié par recristallisation dans un mélange hexane-éther pour conduire à 4 g de produit pur.
Rendement : 70 %
Point de fusion : 98,5-99 ° C

Les exemples 2 à 13 sont obtenus selon la même procédure en utilisant, à l'étape b, le chlorure d'aryle approprié.

### EXEMPLE 2 :

### 1-(4-Chlorobenzyl)-2-(4,5-dihydro[1H]imidazol-2-yl)-4-méthylpipérazine

Rendement : 49 %
Point de fusion : 198-200 °C (Trichlorhydrate dihydraté)

### EXEMPLE 3 :

### 1-(2-Méthoxybenzyl)-2-(4,5-dihydro[1H]imidazol-2-yl)-4-méthylpipérazine

Rendement : 36 %
Point de fusion : 168-170 °C (Trichlorhydrate dihydraté)

### EXEMPLE 4 :

### 1-(3-Méthoxybenzyl)-2-(4,5-dihydro[1H]imidazol-2-yl)-4-méthylpipérazine

Rendement : 38 %
Point de fusion : 78-80 °C (Dichlorhydrate 1,5 hydraté)

### EXEMPLE 5 :

### 1-(2,3-Diméthoxybenzyl)-2-(4,5-dihydro[1H]imidazol-2-yl)-4-méthylpipé-razine

Rendement : 41 %
Point de fusion : 160-162 °C (Dichlorhydrate monohydraté)

### EXEMPLE 6 :

### 1-(2,3-Dichlorobenzyl)-4-méthyl-2-(4,5-dihydro[1H]imidazol-2-yl)pipérazine

Rendement : 58 %
Point de fusion : 158-160 °C (décomposition) (Dichlorhydrate monohydraté)

### EXEMPLE 7 :

### 1-(2,4-dichlorobenzyl)-2-(4,5-dihydro[1H]imidazol-2-yl)-4-méthylpipérazine

Rendement : 60 %
Point de fusion : 167-169 °C (Dichlorhydrate monohydraté)

### EXEMPLE 8 :

### 1-Benzyl-2-(4,5-dihydro[1H]imidazol-2-yl)-4-méthylpipérazine

Rendement : 55 %
Point de fusion : 166-168 °C (Dichlorhydrate 0,75 hydraté)

### EXEMPLE 9 :

### 1-(2-Fluorobenzyl)-2-(4,5-dihydro[1H]imidazol-2-yl)-4-méthylpipérazine

Rendement : 37 %
Point de fusion : 160-162 °C (Dichlorhydrate dihydraté)

### EXEMPLE 10 :

### 1-(2,6-Dichlorobenzyl)-2-(4,5-dihydro[1H]imidazol-2-yl)-4-méthylpipérazine

Rendement : 43 %
Point de fusion : 182-184 °C (Dichlorhydrate monohydraté)

### EXEMPLE 11 :

### 1-(3,4,5-Triméthoxybenzyl)-2-(4,5-dihydro[1H]imidazol-2-yl)-4-méthylpipé-razine

Rendement : 45 %
Point de fusion : 185-187 °C (Dichlorhydrate monohydraté)

### EXEMPLE 12 :

### 1-(2-Méthylbenzyl)-2-(4,5-dihydro[1H]imidazol-2-yl)-4-méthylpipérazine

Rendement : 51 %
Point de fusion : 156-158 °C (Dichlorhydrate 1,5 hydraté)

### EXEMPLE 13 :

### 1-(2-Trifluorométhylbenzyl)-2-(4,5-dihydro[1H]imidazol-2-yl)-4-méthylpipérazine

Rendement : 45 %
Point de fusion : 163-165 °C (Dichlorhydrate monohydraté)

### EXEMPLE 14 :

### 1-Méthyl-2-(4,5-dihydro[1H]imidazol-2-yl)-4-(2-chlorobenzyl)pipérazine

Une solution de 23 g du composé obtenu à la préparation B et de 55 ml de triéthylamine dans 300 ml de toluène est chauffée à reflux. 16,1 g (0,1 mole) de chlorure de 2-chlorobenzyle, dissous dans 200 ml de toluène, sont ajoutés goutte à goutte. Après addition, le chauffage est maintenu pendant 3 heures. Le mélange est refroidi et la phase organique traitée de manière habituelle. Le résidu est purifié par chromatographie sur colonne de gel de silice (éluant éther de pétrole/éther 3:7, puis éther), afin d'isoler 10 g de composé monobenzylé qui est ensuite traité selon le procédé décrit dans l'exemple 1, étapes c et suivantes. La purification par flash chromatographie permet également d'isoler 11 g de 1,4-[di-(2-chlorobenzyl)]-2-éthoxycarbonylpipérazine utilisée pour l'obtention du composé de l'exemple 16.
Rendement : 62 % (à partir de la 1-méthyl-4-(2-chlorobenzyl)-2-éthoxycarbonylpipérazine).
Point de fusion : 140-142 °C (décomposition) (Trichlorhydrate monohydraté)

### EXEMPLE 15 :

### 1-Méthyl-2-(4,5-dihydro[1H]imidazol-2-yl)-4-(2,4-dichlorobenzyl)pipérazine

En procédant comme pour l'exemple 14 mais en remplaçant le chlorure de 2-chlorobenzyle par le chlorure de 2,4-dichlorobenzyle, le produit du titre est obtenu.

### EXEMPLE 16 :

### 1,4-[di-(2-Chlorobenzyl)]-2-(4,5-dihydro[1H]imidazol-2-yl)pipérazine

Ce composé est obtenu selon le procédé décrit dans l'exemple 14 en recueillant lors de la purification sur colonne de gel de silice le composé disubstitué.
Rendement : 67 % (à partir de la 1,4-[di-(2-chlorobenzyl)]-2-éthoxycarbonylpipérazine
Point de fusion : 86-90 °C (Trichlorhydrate dihydraté)

En opérant de la même manière que pour l'exemple 16 et en remplaçant dans l'exemple 14 le chlorure de 2-chlorobenzyle par les chlorures de benzyle appropriés, on obtient les composés des exemples 17 et 18 suivants :

### EXEMPLE 17 :

### 1,4-[Di-(2,4-dichlorobenzyl)]-2-(4,5-dihydro[1H]imidazol-2-yl)pipérazine

Rendement : 62 %
Point de fusion : 188-190°C (Dichlorhydrate monohydraté)

### EXEMPLE 18 :

### 1,4-Dibenzyl-2-(4,5-dihydro[1H]imidazol-2-yl)pipérazine

### EXEMPLE 19 :

### 1,4-Dibenzyl-2-[(4,5-dihydro[1H]imidazol-2-yl)méthyl]pipérazine

### - Etape a : 1,4-Dibenzyl-2-hydroxyméthylpipérazine

5 g (131 mmoles) d'hydrure double de lithium et d'aluminium sont agités dans 200 ml de tétrahydrofurane sec et maintenus à 0°C dans un bain de glace. Une solution de 60 g (175 mmoles) de composé obtenu à la préparation A dissous dans 200 ml de tétrahydrofurane anhydre est alors ajoutée goutte à goutte. Le mélange est maintenu sous agitation à 0°C pendant une heure puis à température ambiante pendant 24 heures. L'excès d'hydrure double de lithium et d'aluminium est ensuite décomposé à 0°C par addition d'une solution d'hydroxyde de sodium à 20 %. Après filtration de l'hydroxyde d'aluminium formé, la phase organique est évaporée et le résidu obtenu est cristallisé dans l'hexane et permet d'obtenir 48,3 g de cristaux blancs du produit attendu.
Rendement : 92 %

### - Etape b : 1,4-Dibenzyl-2-chlorométhylpipérazine

A 20 g (67,6 mmoles) de composé obtenu à l'étape précédente dissous dans 200 ml de benzène, sont additionnés goutte à goutte 5,92 ml (81 mmoles) de chlorure de thionyle. Le mélange est agité pendant 3 heures à température ambiante. Après évaporation du benzène, le résidu obtenu est repris au chloroforme, lavé 3 fois à l'eau jusqu'à pH neutre. La phase organique est séchée sur sulfate de magnésium, filtrée et évaporée, le résidu obtenu est chromatographié sur colonne de gel de silice (éluant : éther/éther de pétrole, 10:90). Cette purification conduit à 17 g de produit attendu sous forme d'huile.
Rendement : 80 %

### - Etape c : 1,4-Dibenzyl-2-cyanométhylpipérazine

Un mélange de 4,57 g (70,2 mmoles) de cyanure de potassium et 10 ml d'eau, sont chauffés à reflux. Après dissolution, 17 g (54 mmoles) du chlorure obtenu à l'étape b dissous dans 10 ml d'éthanol sont ajoutés lentement. A la fin de l'addition le mélange est chauffé à reflux pendant 3 heures. Après évaporation du solvant, le mélange est repris au chloroforme, lavé à l'eau jusqu'à pH neutre. La phase chloroformique est séchée sur sulfate de magnésium, filtrée et évaporée. Le résidu obtenu est cristallisé dans l'hexane et conduit à 13,2 g de cristaux blancs.
Rendement : 80 %

### - Etape d : 1,4-Dibenzyl-2-éthoxycarbonylméthylpipérazine

A 13,2 g (43,3 mmoles) du composé obtenu à l'étape c sont ajoutés 10 g d'acide sulfurique concentré et 30 g d'éthanol absolu. Le mélange est chauffé à reflux (120°C) pendant 3 heures. L'excès d'éthanol est distillé et le résidu obtenu est repris avec une solution aqueuse de carbonate de sodium et extrait au chloroforme. La phase organique est séchée sur sulfate de magnésium, filtrée et évaporée. Le résidu obtenu est chromatographié sur colonne de gel de silice (éluant : éther/éther de pétrole 10:90). Cette purification conduit à 11,4 g du composé attendu sous forme d'huile.
Rendement : 75 %

### - Etape e : 1,4-Dibenzyl-2-[(4,5-dihydro[1H]imidazol-2-yl)méthyl]pipérazine

Procédure identique à celle utilisée pour l'exemple 1, étape d, à partir de l'ester obtenu à l'étape précédente.
Rendement : 47 %
Point de fusion : 176-178°C (Trichlorhydrate dihydraté)

### EXEMPLE 20 :

### 1-(2-Méthoxybenzyl)-2-[(4,5-dihydro[1H]imidazol-2-yl)méthyl]-4-méthylpipérazine

Composé obtenu selon une manière identique à celle décrite pour l'exemple 3 à partir de l'ester obtenu dans l'exemple 19, étape d.

### EXEMPLE 21 :

### 1-(2-Chlorobenzyl)-2-(4,5-dihydro[1H]imidazol-2-yl)-4-n-propylpipérazine

Le composé obtenu à l'exemple 1, étape b est traité dans l'acétone par le chlorure de n-propyle en présence d'iodure de potassium et de carbonate de potassium. La 1-(2-chlorobenzyl)-4-n-propyl-2-éthoxycarbonylpipérazine ainsi obtenu est ensuite traitée comme décrit à l'exemple 1, étape d.
Rendement : 65 %
Point de fusion : 160-163°C (décomposition) (Trichlorhydrate monohydraté)

### EXEMPLE 22 :

### 1-(2-Chlorobenzyl)-2-(4,5-dihydro[1H]imidazol-2-yl)-4-isopropylpipérazine

Procédé identique à celui décrit dans l'exemple précédent, en remplaçant le chlorure de n-propyle par le bromure d'isopropyle.
Rendement : 54 %
Point de fusion : 156°C (décomposition) (Dichlorhydrate monohydraté)

### EXEMPLE 23 :

### 1-(2-Chlorobenzyl)-2-(4,5-dihydro[1H]imidazol-2-yl)-4-éthylpipérazine

Composé obtenu selon un procédé identique en remplaçant le chlorure de n-propyle par le bromure d'éthyle.
Rendement : 30 %
Point de fusion : 163°C (décomposition) (Dichlorhydrate)

Les deux exemples suivants sont obtenus selon le même procédé que celui décrit dans l'exemple 14 en remplaçant à chaque fois la méthylation par le formol par une alkylation avec l'halogénure d'alkyle approprié selon la méthode décrite à l'exemple 21.

### EXEMPLE 24 :

### 1-Ethyl-2-(4,5-dihydro[1H]imidazol-2-yl)-4-(2-chlorobenzyl)pipérazine

Rendement : 64 %
Point de fusion : 139°C

### EXEMPLE 25 :

### 1-Isopropyl-2-(4,5-dihydro[1H]imidazol-2-yl)-4-(2-chlorobenzyl)pipérazine

Rendement : 58 %
Point de fusion : 159°C (Trichlorhydrate)

### EXEMPLE 26 :

### 1-(2-Chlorobenzyl)-2-(4,5-dihydro[1H]imidazol-2-yl)pipérazine

Mode opératoire identique à celui décrit dans l'exemple 1, en omettant l'étape c.
Rendement : 28 %
Point de fusion : 161°C (décomposition) (Trichlorhydrate monohydraté)

### EXEMPLE 27 :

### 1-(Dicyclopropylméthyl)-2-(4,5-dihydro[1H]imidazol-2-yl)-4-méthylpipérazine

### - Etape a : N-Méthyl-N-benzyl-2-aminoéthanamine

110 g (0,5 mole) du chlorure obtenu à l'étape b de la préparation C sont mis en suspension dans 11 d'eau saturée d'ammoniac. Le mélange est agité 3 jours à température ambiante le solvant est partiellement évaporé sous vide et la solution est rendue basique par addition d'hydroxyde de sodium. L'amine est extraite au dichlorométhane et après séchage sur sulfate de magnésium et évaporation de solvant, le produit est purifié par distillation. On isole 49 g du composé attendu.
Rendement : 60 %
Point d'ébullition : (2000 Pa) 152-154°C

### - Etape b : N-Méthyl-N-benzyl-2-(N'-dicyclopropylméthylamino)éthanamine

Un mélange constitué de 8,2 g (0,05 mole) de composé obtenu à l'étape a, 5,5 g (0,05 mole) de dicyclopropylcétone et de 50 ml de cyclohexane est chauffé pendant 15 jours. Le solvant est évaporé sous vide et on ajoute au résidu 50 ml de méthanol sec et froid, puis 1,9 g (0,05 mole) de borohydrure de sodium. Après 15 heures d'agitation à température ambiante la solution est versée dans 50 ml d'eau contenant 2 g (0,05 mole) de soude et saturée de chlorure de sodium puis extraite à l'hexane. Le produit est purifié sur colonne de silice (éluant : dichlorométhane pur puis dichlorométhane-méthanol, 97:3). On obtient 6 g de composé attendu.
Rendement : 47 %

### - Etape c : N-Méthyl-2-(N'-dicyclopropylméthylamino)éthanamine

5,8 g de composé obtenu à l'étape b, dans 30 ml d'éthanol absolu et 100 mg de palladium sur charbon à 10 %, sont agités et chauffés à 45 °C sous atmospère d'hydrogène pendant 3 heures. Le solvant est évaporé. Après traitement habituel de la phase organique le produit brut attendu est purifié par distillation.
Rendement : 85 %
Point d'ébullition : (6,66 Pa) 48-52°C

### - Etape d : 1-Dicyclopropylméthyl-2-éthoxycarbonyl-4-méthylpipérazine

Le mélange constitué de 4,4 g (0,026 mole) de diamine obtenue précédemment, 10 ml de triéthylamine et 50 ml de benzène, est chauffé à reflux, puis sont ajoutés 6,4 g (0,0246 mole) de 2,3 dibromopropionate de méthyle dans 20 ml de benzène. Après 3 heures de chauffage à reflux, le mélange est refroidi et filtré. La solution est lavée à l'eau et après traitement habituel le résidu est purifié par chromatographie sur colonne de silice. (Eluant : éther). 5,1 g de composé pur attendu sont obtenus.
Rendement : 78 %

### - Etape e : 1-(Dicyclopropylméthyl)-2-(4,5-dihydro[1H]imidazol-2-yl)-4-méthylpipérazine

L'ester obtenu à l'étape d précédente est engagé dans la réaction décrite à l'exemple 1, étape d.
Rendement : 50 %
Point de fusion : 98°C

### EXEMPLE 28 :

### 1,4-Diisopropyl-2-(4,5-dihydro[1H]imidazol-2-yl)pipérazine

En faisant réagir la 2-éthoxycarbonylpipérazine avec 2,2 équivalents de bromure d'isopropyle dans les conditions de l'exemple 21, puis en traitant le composé obtenu dans les conditions de l'exemple 1, étape d, le produit titre est obtenu.
Rendement : 35 %
Point de fusion : 145°C (décomposition) (Trichlorhydrate dihydraté)

### EXEMPLE 29:

### 1 -(2-Chlorobenzyl)-2-(4,5-dihydro[1H]oxazol-2-yl)4-méthylpipérazine

### - Etape a : 2-[(Tétrahydropyran-2-yl)oxy]éthylamine

Une mole de 2-aminoéthanol est mise en présence de deux équivalents de chlorure de benzyle, et de triéthylamine en solution dans l'éthanol. Le chlorhydrate d'hydroxy-amine ainsi protégée est soumise à l'action de dihydropyrane en solution dans le dichlorométhane, puis l'amine est déprotégée par action d'hydrogène gazeux en présence de catalyseur (palladium sur charbon).

### - Etape b : 1-(2-Chlorobenzyl)-2-[(2-hydroxyéthylamino)carbonyl]-4-méthylpipérazine

7,3 g (0,05 mole) du composé obtenu à l'étape a sont dilués dans 80 ml de toluène anhydre. A la solution dégazée et refroidie par un bain de glace, 25 ml (0,05 mole) de solution 2M de triméthylaluminium dans du toluène sont ajoutés. Après addition, le mélange est ramené à température ambiante et 10 g (0,034 mole) du composé obtenu dans l'exemple 1, étape c, sont ajoutés. Le milieu réactionnel est chauffé 4 heures à reflux, puis hydrolysé avec 100 ml de solution méthanol/eau (8:2). Le précipité qui se forme est filtré, les solvants sont évaporés sous vide et le résidu dissous dans du dichlorométhane. La phase organique est lavée à l'eau, séchée sur sulfate de magnésium et le solvant à nouveau évaporé sous vide. Le produit brut est repris par du méthanol et une solution saturée d'acide chlorhydrique gazeux. Le mélange est agité 2 heures à température ambiante, le solvant évaporé sous vide et le sel dissous dans de l'eau. La phase aqueuse est extraite au dichlorométhane puis neutralisée par du carbonate de sodium. Après traitement habituel on obtient 7,3 g du composé attendu sous forme d'une huile.
Rendement : 69 %

### - Etape c : 1-(2-Chlorobenzyl)-2-[(2-chloro-éthylamino)carbonyl]-4-méthylpipérazine

A 7 g (0,027 mole) de composé obtenu à l'étape précédente dans 50 ml de chloroforme refroidis dans un bain de glace, on ajoute goutte à goutte 2,7 ml (0,037 mole) de chlorure de thionyle dilué dans 10 ml de chloroforme. Le mélange est agité 15 heures à température ambiante puis versé dans 60 ml de solution 1M de carbonate de sodium. Après traitement habituel, le produit est purifié sur colonne de silice, (éluant : éther/éther de pétrole, 1:1), puis éther). Le composé attendu ainsi purifié est recristallisé dans un mélange hexane-éther. 4,5 g de cristaux jaunes pâles sont isolés.
Rendement : 50 %
Point de fusion : 74-75°C

### - Etape d : 1-(2-Chlorobenzyl)-2-(4,5-dihydro[1H]oxazol-2-yl)-4-méthylpipérazine

Une solution chaude de 0,260 g (0,068 mole) d'hydroxyde de sodium dans 6 ml d'éthanol à 80 %, est ajoutée goutte à goutte à une solution chaude de 2,2 g (0,067 mole) du chlorure obtenu précédemment dans 15 ml d'éthanol. Le mélange est chauffé 30 minutes à reflux. On évapore le solvant sous vide et le résidu est repris par l'éther. Après traitement habituel, on isole par cristallisation dans l'héxane 1,7 g de cristaux blancs.
Rendement : 85 %
Rendement global : 29 %
Point de fusion : 96,8°C

### EXEMPLE 30 :

### 1-Méthyl-4-(2-chlorobenzyl)-2-(4,5-dihydro[1H]oxazol-2-yl)pipérazine

Mode opératoire identique à celui décrit pour l'exemple 14 jusqu'à la formation de l'ester puis, création du radical oxazolinyle comme décrit dans l'exemple 29.

### EXEMPLE 31 :

### 1-Dicyclopropyl-méthyl-4-méthyl-2-(4,5-dihydro[1H]oxazol-2-yl)pipérazine

Mode opératoire identique à celui décrit pour l'exemple 30, à partir du mode opératoire de l'exemple 27.

### EXEMPLE 32 :

### 1,4-Dibenzyl-2-[(4,5-dihydro[1H]oxazol-2-yl)méthyl]pipérazine

Mode opératoire identique à celui décrit pour l'exemple 30, à partir du mode opératoire de l'exemple 19.

### EXEMPLE 33 :

### 1,4-Dibenzyl-2-[2-(4,5-dihydro[1H]imidazol-2-yl)éthyl]pipérazine

L'ester obtenu à l'étape d de l'exemple 19 est à nouveau engagé dans les étapes a, b, c, d et e de l'exemple 19 pour conduire au produit du titre.

### EXEMPLE 34 :

### 1-Méthyl-4-(2-chlorobenzyl)-2-[2-(4,5-dihydro[1H]oxazol-2-yl)éthyl] pipérazine

Comme obtenu selon le mode opératoire décrit à l'exemple 33 à partir de l'ester obtenu comme intermédiaire dans l'exemple 30.

### EXEMPLE 35 :

### 1,4-Dibenzyl-2-[(1-phénoxycarbonyl)-4,5-dihydro[1H]imidazol-2-yl]pipérazine

Composé obtenu selon le mode opératoire de l'exemple 16 en utilisant, afin de former le radical imidazolinyle, la N-phényloxycarbonyléthylène diamine en lieu et place de l'éthylène diamine.
Rendement : 75 %
Point de fusion : 129-131 °C

### EXEMPLE 36 :

### 1,4-Dibenzyl-2-[(N-méthyl)-4,5-dihydro[1H]imidazol-2-yl]pipérazine

Composé obtenu selon le mode opératoire de l'exemple 16 en utilisant, afin de former le radical imidazolinyle, la N-méthyléthylène diamine en lieu et place de l'éthylène diamine.

### EXEMPLE 37 :

### 1-Phényl-4-(2-chlorobenzyl)-2-(4,5-dihydro[1H]imidazol-2-yl)pipérazine

Ce composé est obtenu selon l'exemple 1 en remplaçant la 2-éthoxycarbonylpipérazine par la 1-phényl-2-éthoxycarbonylpipérazine obtenue à partir de N-phényl-N'-benzyléthylène diamine.

### EXEMPLE 38 :

### 1-Cyclohexyl-4-(2-chlorobenzyl)-2-(4,5-dihydro[1H]imidazol-2-yl)pipérazine

Ce composé est obtenu selon la méthode décrite dans l'exemple 24 en remplaçant le bromure d'éthyle par le bromure de cyclohexyle.

### EXEMPLE 39 :

### 1-Benzyl-4-méthyl-2-(1,4,5,6-tétrahydro[1H]pyrimidin-2-yl)pipérazine

Composé obtenu selon le mode opératoire décrit dans l'exemple 8, en remplaçant l'éthylène-diamine par le 1,3-diaminopropane.

### EXEMPLE 40 :

### 1-(2,4-Dichlorobenzyl)-2-(4,5-dihydro[1H]imidazol-2-yl)-2,4-diméthylpipérazine

En procédant comme pour l'exemple 7, mais en remplaçant dans la dernière étape, la 4-méthyl-2-éthoxycarbonyl-1-(2,4-dichlorobenzyl)pipérazine par la 2,4-diméthyl-2-éthoxycarbonyl-1-(2,4-dichlorobenzyl)pipérazine (préparation C), le composé attendu est obtenu.

### EXEMPLE 41 :

### 1,4-Diisopropyl-2-(4,5-dihydro[1H]imidazol-2-yl)-2-méthylpipérazine

Composé obtenu selon une méthode similaire à celle décrite à l'exemple 40.

### EXEMPLE 42 :

### 1-(2,4-Dichlorobenzyl)-4-méthyl-2-[(N-méthyl)-4,5-dihydro[1H]imidazol-2-yl]pipérazine

Composé obtenu selon un mode opératoire similaire à celui décrit pour l'exemple 36.
Point de fusion : 61-63°C (Chlorhydrate)

### EXEMPLE 43 :

### 1-(2,4-dichlorobenzyl)-2-(5-méthyl-4,5-dihydro[1H]imidazol-2-yl)4-méthylpipérazine

Composé obtenu selon un mode opératoire identique à celui de l'exemple 7, mais en remplaçant dans la dernière étape, l'éthylènediamine par le 1,2-diaminopropane. Point de fusion : 165-167°C (Chlorhydrate) (décomposition)

### EXEMPLE 44 :

### 1-(2,4-dichlorobenzyl)-2-(3,4,5,6-tétrahydro[3H]pirimidin-2-yl)-4-méthylpipérazine

Composé obtenu selon le mode opératoire décrit à l'exemple 7 mais en remplaçant dans la dernière étape l'éthylènediamine par le 1,3-diaminopropane.

### EXEMPLE 45 :

### 2-(4,5-dihydro[1H]imidazol-2-yl)-1,4-diisobutylpipérazine

Composé obtenu selon le mode opératoire décrit à l'exemple 28 en remplaçant le bromure d'isopropyle par le bromure d'isobutyle.
Point de fusion : 142-144°C (Trichlorhydrate monohydraté)

### ETUDE PHARMACOLOGIQUE

### EXEMPLE A : Mise en évidence de l'activité antidiabétique

Effet des produits de l'invention sur la tolérance au glucose chez le rat rendu intolérant au glucose par une faible dose de streptozotocine.

### 1) Protocole expérimental

Des rats Wistar mâles âgés de 11 semaines sont traités avec de la streptozotocine (35 mg/kg i.p.) afin de détruire partiellement les cellules β-pancréatiques. Seuls les animaux présentant une intolérance au glucose semblable à celle d'un diabète de type II ont été utilisés dans la suite des expériences (THIBAULT et al., Endocrinology, (1992), 130 (5), 2521-2527).

Les animaux ainsi sélectionnés sont ensuite prétraités (6 par dose) par voie i.p., p.o. ou i.v. par les composés à tester, 10 minutes avant l'administration par voie i.v. de glucose (0,5 g/kg de poids corporel). Les paramètres représentatifs de métabolisme glucidique sont mesurés en comparaison avec des animaux témoins.

Le mode opératoire sur chaque rat peut être schématisé ainsi :

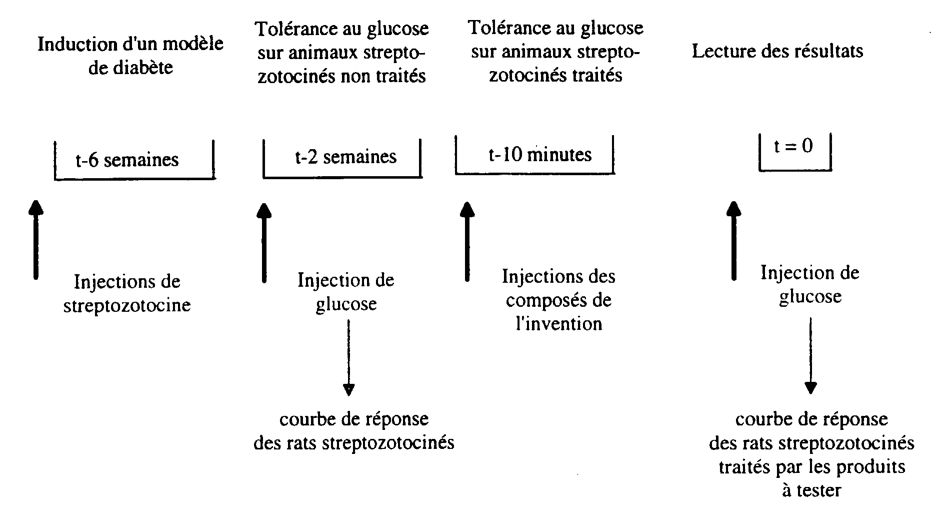

### 2) Résultats

Des échantillons sanguins sont prélevés 0, 5, 10, 15, 20, 25 et 30 minutes après l'injection de glucose. Les concentrations sanguines de glucose sont alors déterminées. La tolérance au glucose est alors objectivée par :
- la détermination du ΔG qui représente l'intégration des variations de la glycémie au dessus du niveau basal pendant la durée du test. Une diminution du ΔG après administration d'un composé à tester est signe d'activité anti-diabétique.
- la détermination du coefficient K qui représente la pente de décroissance des concentrations en glucose et rendant compte de l'assimilation du glucose. Plus la valeur du coefficient K est élevée, meilleure est l'activité du produit testé.

Les produits de l'invention se montrent remarquablement actifs dans ce test. En effet, ils sont responsables d'une diminution dose-dépendante du ΔG déterminé, associée parallèlement à une augmentation du coefficient K.

Les produits de l'invention les plus actifs ramènent les valeurs de glycémie au niveau des valeurs d'animaux non diabétiques.

Les résultats obtenus sont exprimés dans le tableau suivant :

### Mise en évidence de l'activité anti-diabétique

| **PRODUIT** | **DOSE** (µM/kg) | **VOIE** | **ΔG** (g/l) | **K** |
|---|---|---|---|---|
| *EXEMPLE 1* | 100 | i.p. | 18,24 | 2,56 |
| | 0 | | 22,82 | 1,64 |
| | 100 | i.v. | 8,54 | 2,88 |
| *EXEMPLE 7* | 100 | i.p. | 12,83 | 3,25 |
| | 0 | | 20,11 | 1,53 |
| | 100 | p.o. | 17,48 | 1,56 |
| | 0 | | 23,43 | 1,1 |
| *EXEMPLE 13* | 100 | i.p. | 14,39 | 2,32 |
| | 0 | | 20,88 | 1,56 |
| *EXEMPLE 14* | 100 | i.p. | 17,16 | 2,96 |
| | 30 | i.p. | 20,26 | 2,26 |
| | 0 | | 23,60 | 1,19 |
| | 100 | i.v. | 6,00 | 3,27 |
| *EXEMPLE 19* | 100 | i.p. | 18,60 | 2,26 |
| | 0 | | 22,02 | 1,28 |
| *EXEMPLE 25* | 100 | i.p. | 12,42 | 2,09 |
| | 0 | | 20,03 | 0,9 |
| *EXEMPLE 27* | 100 | i.p. | 13,76 | 1,96 |
| | 0 | | 20,61 | 1,36 |
| *EXEMPLE 28* | 100 | i.p. | 9,95 | 1,81 |
| | 0 | | 19,23 | 1,21 |
| *EXEMPLE 42* | 100 | i.p. | 16,58 | 2,03 |
| | 0 | | 18,25 | 1,26 |
| *EXEMPLE 43* | 100 | i.p. | 17,56 | 1,97 |
| | 0 | | 19,01 | 1,19 |

### EXEMPLE B : Composition pharmaceutique

### Formule de préparation pour 1000 comprimés dosés à 50 mg.

| | |
|---|---|
| Composé de l'exemple 7 | 50 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 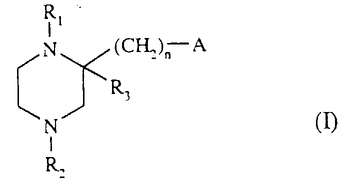 dans laquelle :
- R₁ et R₂ indépendamment l'un de l'autre sont choisis parmi l'hydrogène, les radicaux alkyles, cycloalkyles, cycloalkylalkyles, bicycloalkylalkyles, aryles et arylalkyles, chacun de ces radicaux étant éventuellement substitué,
- R₃ est choisi parmi l'hydrogène et un radical alkyle,
- n prend indifféremment une valeur choisie parmi 0, 1 et 2,
- A représente le radical 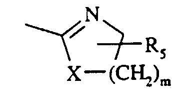 dans lequel
- X représente l'oxygène ou le groupement N-R₄,
- m prend une valeur choisie parmi 1 et 2,
- R₄ est choisi parmi l'hydrogène et les radicaux alkyles, alkoxycarbonyles et aryloxycarbonyles éventuellement substitués, et
- R₅ est choisi par mi l'hydrogène et un radical alkyle,
étant entendu que :
- les termes "alkyle", "alkoxy", "monocycloalkylalkyle", "dicycloalkylalkyle", "arylalkyle" et "alkoxycarbonyle" désignent des radicaux dont la chaîne hydrocarbonée comporte de 1 à 10 atomes de carbone en chaîne droite ou ramifiée,
- le terme "cycloalkyle" désigne des radicaux hydrocarbonés cycliques comportant de 3 à 8 atomes de carbone,
- les termes "aryle", "arylalkyle" et "aryloxycarbonyle" concernent des radicaux dont la partie aromatique est un radical phényle ou un radical naphtyle,
- l'expression "éventuellement substitué" signifie que les radicaux ainsi qualifiés peuvent être substitués par une ou plusieurs entités chimiques choisies parmi les radicaux alkyles, hydroxy, alkoxy, halogéno, halogénoalkyles, polyhalogénoalkyles, nitro, amino, alkylamino et polyalkylamino,
leurs stéréoisomères, N-oxydes et sels d'addition à un acide ou à une base, pharmaceutiquement acceptables.

2. Composés selon la revendication 1, pour lesquels X représente le groupement -N-R₄ et m est égal à 1, leurs stéréoisomères, N-oxydes et sels d'addition à un acide ou à une base, pharmaceutiquement acceptables.

3. Composés selon la revendication 1, pour lesquels X représente l'oxygène et m est égal à 1, leurs stéréoisomères, N-oxydes et sels d'addition à un acide ou à une base, pharmaceutiquement acceptables.

4. Composé selon l'une quelconque des revendications 1 à 2 qui est la 1-(2,4-dichloro benzyl)-2-(4,5-dihydro[1*H*]imidazol-2-yl)-4-méthylpipérazine, ses stéréoisomères, N-oxydes et sels d'additions à un acide, pharmaceutiquement acceptables.

5. Composé selon l'une quelconque des revendications 1 à 2 qui est la 1-méthyl-2-(4,5-dihydro[1*H*]imidazol-2-yl)-4-(2-chlorobenzyl)pipérazine, ses stéréoisomères, N-oxydes et sels d'additions à un acide, pharmaceutiquement acceptables.

6. Composé selon l'une quelconque des revendications 1 à 2 qui est la 1-méthyl-2-(4,5-dihydro[1*H*]imidazol-2-yl)-4-(2,4-dichlorobenzyl)pipérazine, ses stéréoisomères, N-oxydes et sels d'additions à un acide, pharmaceutiquement acceptables.

7. Composé selon l'une quelconque des revendications 1 à 2 qui est la 1,4-dibenzyl-2-[(4,5-dihydro[1*H*]imidazol-2-yl)méthyl]pipérazine, ses stéréoisomères, N-oxydes et sels d'additions à un acide, pharmaceutiquement acceptables.

8. Composé selon l'une quelconque des revendications 1 et 3 qui est la 1-méthyl-4-(2-chlorobenzyl)-2-(4,5-dihydro[1*H*]oxazol-2-yl)pipérazine, ses stéréoisomères, N-oxydes et sels d'additions à un acide, pharmaceutiquement acceptables.

9. Composé selon l'une quelconque des revendications 1 et 3 qui est la 1,4-dibenzyl-2-[(4,5-dihydro[1*H*]oxazol-2-yl)méthyl]pipérazine, ses stéréoisomères, N-oxydes et sels d'additions à un acide, pharmaceutiquement acceptables.

10. Composé selon l'une quelconque des revendications 1 à 2 qui est la 1,4-diisopropyl-2-(4,5-dihydro[1*H*]imidazole-2-yl)pipérazine, ses stéréoisomères, N-oxydes et sels d'additions à un acide, pharmaceutiquement acceptables.

11. Procédé de préparation des composés de formule (I) selon la revendication 1,caractérisé en ce que :
a) - soit, le composé de formule (II) :
C₆H₅-CH₂-NH-CH₂-CH₂-NH-CH₂-C₆H₆ (II)
est porté à reflux dans un solvant aromatique en présence de triéthylamine avec le dibromure de formule (III) : 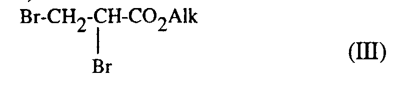 dans laquelle Alk représente un radical alkyle linéaire ou ramifié comportant de 1 à 4 atomes de carbone,
pour conduire au composé de formule (IV) : 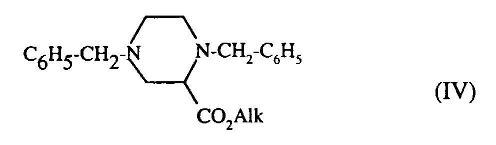 dans laquelle Alk est tel que défini précédemment,
qui est ensuite débenzylé, par action d'hydrogène en présence de catalyseur, pour conduire au composé de formule (V) : 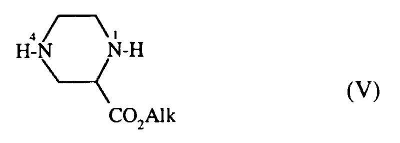 dans laquelle Alk est tel que défini précédemment,
qui est transformé ensuite en composé de formule (Va), (Vb) ou (Vc) : 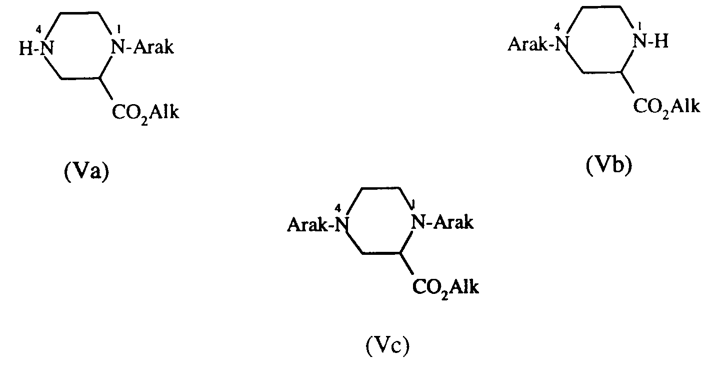 où Arak représente un radical arylalkyle éventuellement substitué comme défini précédemment, et Alk est tel que défini précédemment,
- pour le composé de formule (Va) :
après protection de l'atome d'azote 4 par le chlorure de triphénylméthyle en solvant approprié, en présence de triéthylamine, par traitement par Arak-Cl en présence de carbonate de potassium et d'une quantité catalytique d'iodure de potassium, en solvant approprié, puis déprotection de l'atome d'azote 4, par le chlorure d'hydrogène gazeux en solvant adéquat,
- pour les composés de formules (Vb) et (Vc) :
par traitement du composé de formule (V) directement par Arak-Cl dans le toluène à reflux en présence de triéthylamine, puis séparation par chromatographie sur colonne de gel de silice des dérivés mono- et disubstitués,
b) - soit, le composé de formule (VI) : 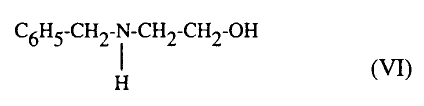 est éventuellement traité par une cétone ou un aldéhyde, en milieu acide et alcoolique, la cétone ou l'aldéhyde choisi étant l'homologue du radical R'₁ (R'₁ ayant la même définition que R₁, l'hydrogène excepté) que l'on souhaite greffer sur l'atome d'azote, afin d'obtenir le composé de formule (VII) : 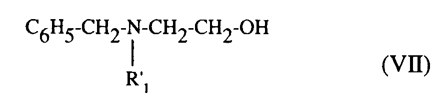 dans laquelle R'₁ est tel que défini précédemment,
composés de formules (VI) et (VII), qui, traités par un agent de chloration, fournissent les dérivés chlorés correspondants, puis sous action d'une solution aqueuse saturée d'ammoniaque conduisent au composé de formule (VIII) : 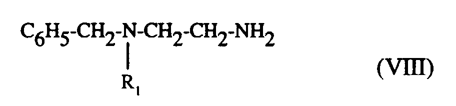 dans laquelle R₁ est tel que défini précédemment,
pour être éventuellement traité ensuite, comme le composé de formule (VI) par l'homologue du radical R'₂, (R'₂ possédant la même définition que R₂, l'hydrogène excepté), sous forme d'aldéhyde ou de cétone, et conduire, après hydrogénation de la double liaison formée, à la diamine de formule (IX) : 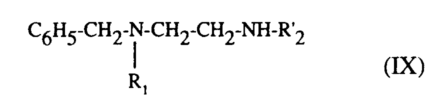 dans laquelle R₁ et R'₂ sont tels que définis précédemment,
les composés de formules (VIII) et (IX) étant alors débenzylés selon le procédé décrit pour le composé de formule (IV) puis traités par le dibromure de formule (III) décrit précédemment pour donner après séparation des éventuels isomères par chromatographie et/ou cristallisation, le composé de formule (X) : 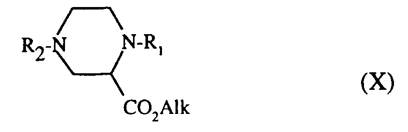 dans laquelle R₁, R₂ et Alk sont tels que définis précédemment,
c) - soit, le composé de formule (VI) : 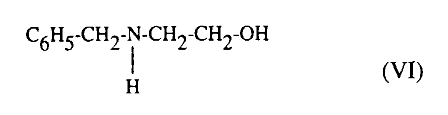 est éventuellement traité par une cétone ou un aldéhyde, en milieu acide et alcoolique, la cétone ou l'aldéhyde choisi étant l'homologue du radical R'₂ (R'₂ ayant la même définition que R₂, l'hydrogène excepté) que l'on souhaite greffer sur l'atome d'azote, afin d'obtenir le composé de formule (XI) : 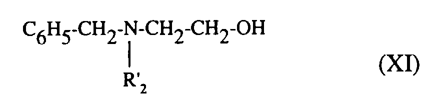 dans laquelle R'₂ est tel que défini précédemment,
composés de formules (VI) et (XI) qui traités par un agent de chloration, fournissent les dérivés chlorés correspondants qui, traités par la benzylamine conduisent à la diamine de formule (XII) : 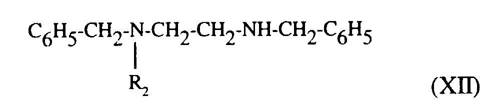 dans laquelle R₂ est tel que défini précémment,
qui par réaction avec un dérivé halogéné de formule (XIII) : 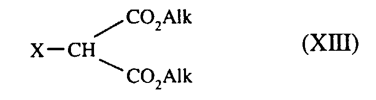 dans laquelle X représente un atome d'halogène et Alk est tel que défini précédemment,
conduit à la diamine de formule (XIV) : 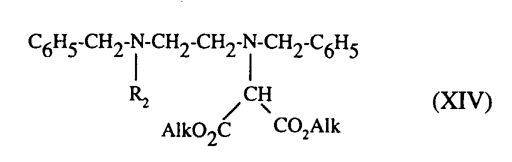 dans laquelle Alk et R₂ sont tels que définis précédemment,
qui après métallation et réaction avec un dérivé halogéné de formule (XV) :
X-R₃ (XV)
dans laquelle X et R₃ sont tels que définis précédemment,
conduit à la diamine de formule (XVI) : 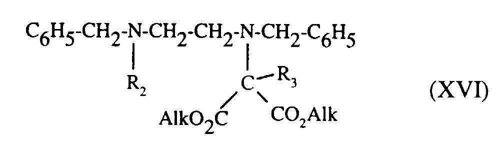 dans laquelle R₂, R₃ et Alk sont tels que définis précédemment,
qui, après débenzylation catalytique et cyclisation, conduit à la pipérazinone de formule (XVII) : 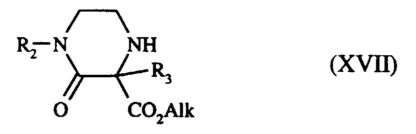 dans laquelle R₂, R₃ et Alk sont tels que définis précédemment,
qui :
- soit par action d'aldéhyde ou de cétone précurseurs du radical R'₁ selon la méthode décrite précédemment,
- soit par alkylation directe avec un dérivé halogéné de formule (XVIII) :
R'₁-X (XVIII)
dans laquelle R'₁ et X sont tels que définis précédemment,
conduit la pipérazinone de formule générale (XIX) : 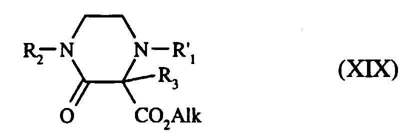 dans laquelle R'₁, R₂, R₃ et Alk sont tels que définis précédemment,
pipérazinones de formules (XIX) et (XVII) qui, par réduction avec des hydrures de bore, conduisent à la pipérazine de formule (XX) : 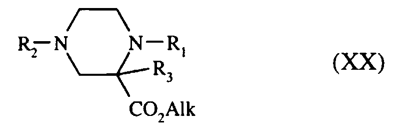 dans laquelle R₁, R₂, R₃ et Alk sont tels que définis précédemment,
les amines secondaires des composés de formules (V), (Va) et (Vb) pouvant également être substituées :
- soit par action d'aldéhydes ou de cétones précurseurs des radicaux R'₁ ou R'₂ selon la méthode décrite précédemment,
- soit par alkylation directe avec un dérivé halogéné de formule (XXI) :
R-X (XXI)
dans laquelle R a la même définition que celle de R'₁ ou R'₂ et X représente un halogène,
avec, dans le cas des composés de formule (V), protection éventuelle au préalable de l'atome d'azote le plus réactif par un groupement triphénylméthyle, comme indiqué pour l'obtention du composé de formule (Va),
les composés de formules (V), (Va), (Vb), (Vc), leurs éventuels produits de substitutions tels qu'envisagés précedemment, ainsi que les composés de formule (X) faisant également partie des composés de formule (XX),
composés de formules (XX) qui peuvent éventuellement être transformés en leurs homologues supérieurs de formule (XXII) : 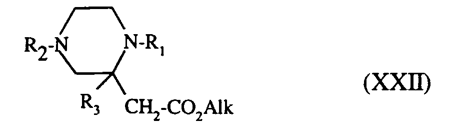 dans laquelle R₁, R₂, R₃ et Alk sont tels que définis précédemment,
par traitement par un agent de réduction, afin de transformer la fonction ester en fonction alcool, puis chloration, suivie d'un traitement par un cyanure d'alcalino-terreux, et finalement transformation du nitrile ainsi obtenu en ester sous l'action d'un alcool en milieu acide,
composé de formule (XXII), qui, éventuellement soumis au même traitement, conduit à l'homologue de formule (XXIII) : 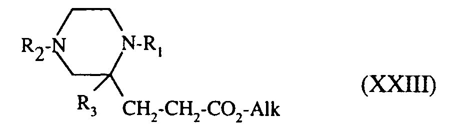 dans laquelle R₁, R₂, R₃ et Alk sont tels que définis précédemment,
l'ensemble des composés de formule (XX), (XXII) et (XXIII) formant l'ensemble des composés de formule (XXIV) : 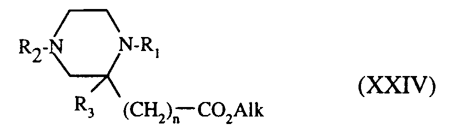 dans laquelle R₁, R₂, R₃, Alk et n sont tels que définis précédemment,
qui sont soumis :
**soit:** a) à l'action d'une diamine de formule (XXV) : 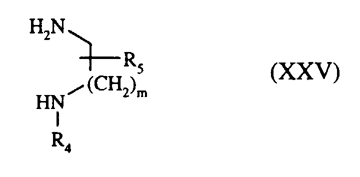 dans laquelle R₄, R₅ et m sont tels que définis précédemment,
en présence d'un trialkylaluminium, en solvant aprotique anhydre, pour conduire au composé de formule (Ia) 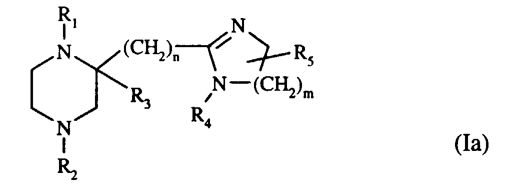 dans laquelle R₁, R₂, R₃, R₄, R₅, n et m sont tels que définis précédemment,
**soit** : b) à l'action du composé de formule (XXVI) : 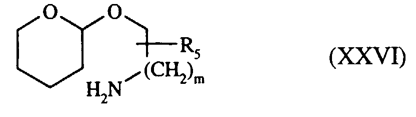 dans laquelle R₅ et m sont tels que définis précédemment,
composé obtenu à partir de l'α-ω-hydroxy-amine correspondante, dont la fonction amine a été préalablement protégée en N,N-dibenzyl-α-ω-hydroxy-amine, sous l'action du chlorure de benzyle, puis protection de la fonction alcool par le dihydropyrane, puis hydrogénolyse en présence de palladium sur charbon afin de libérer la fonction amine,
composé de formule (XXVI) réagissant sur le composé de formule (XXIV) dans les mêmes conditions que le composé de formule (XXV) pour conduire au composé de formule (XXVII) : 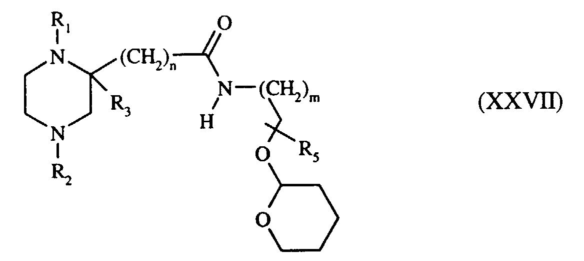 dans laquelle R₁, R₂, R₃, R₅, n et m sont tels que définis précédemment,
composé, dont la fonction alcool est libérée en milieu acide alcoolique, puis transformée en chlorure correspondant sous l'action de chlorure de thionyle pour permettre la cyclisation souhaitée, en milieu basique, et obtenir le composé de formule (Ib) : 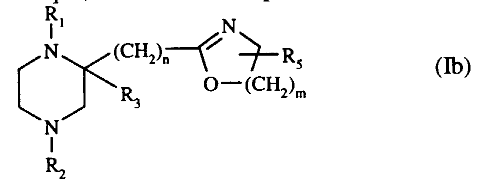 dans laquelle R₁, R₂, R₃, R₅, n et m sont tels que définis précédemment,
l'ensemble des composés de formules (Ia) et (Ib) formant l'ensemble des composés de formule (I), qui sont purifiés et éventuellement séparés en leurs stéréoisomères par une technique classique de séparation et qui sont, si on le souhaite, transformés en leurs N-oxydes ou en leurs sels d'addition à un acide ou à une base, pharmaceutiquement acceptables.

12. Compositions pharmaceutiques contenant comme principe actif au moins un composé selon l'une quelconque des revendications 1 à 10, seul ou en combinaison avec un ou plusieurs véhicules inertes, non toxiques et pharmaceutiquement acceptables.

13. Compositions pharmaceutiques selon la revendication 12 augmentant la tolérance au glucose chez des sujets diabétiques non-insulino-dépendants et utile dans le traitement dudit diabète.

## Patentansprüche

1. Verbindungen der Formel (I): 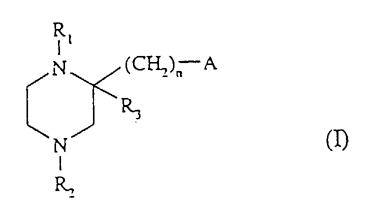 in der:
- R₁ und R₂ unabhängig voneinander aus Wasserstoff, Alkylgruppen, Cycloalkylgruppen, Monocycloalkylalkylgruppen, Dicycloalkylalkylgruppen, Arylgruppen und Arylalkylgruppen ausgewählt sind, wobei jede dieser Gruppen gegebenenfalls substituiert sein kann,
- R₃ aus Wasserstoff und einer Alkylgruppe ausgewählt ist,
- n unabhängig einen Wert ausgewählt aus 0, 1 und 2 darstellt,
- A die Gruppe 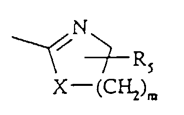 bedeutet, in der
- X Sauerstoff oder die Gruppe N-R₄ darstellt,
- m einen Wert ausgewählt aus 1 und 2 bedeutet,
- R₄ aus Wasserstoff und gegebenenfalls substituierten Alkyl-, Alkoxycarbonyl- und Aryloxycarbonyl-gruppen ausgewählt ist, und
- R₅ aus Wasserstoff und einer Alkylgruppe ausgewählt ist,
mit der Magabe, daß
- die Begriffe "Alkyl", "Alkoxy", "Monocycloalkylalkyl", "Dicycloalkylalkyl", Arylalkyl" und "Alkoxycarbonyl" für Gruppen stehen, deren Kohlenwasserstoffkette 1 bis 10 Kohlenstoffatome in gerader oder verzweigter Kette aufweist,
- der Begriff"Cycloalkyl" für cyclische Kohlenwasserstoffreste mit 3 bis 8 Kohlenstoffatomen steht,
- die Begriffe "Aryl", "Arylalkyl" und "Aryloxycarbonyl" Reste betreffen, deren aromatischer Teil eine Phenylgruppe oder eine Naphthylgruppe ist,
- der Begriff "gegebenenfalls substituiert" bedeutet, daß die in dieser Weise gekennzeichneten Gruppen durch eine oder mehrere chemische Einheiten substituiert sein können, die aus Alkylgruppen, Hydroxylgruppen, Alkoxygruppen, Halogenatomen, Halogenalkylgruppen, Polyhalogenalkylgruppen, Nitrogruppen, Aminogruppen, Alkylaminogruppen und Polyalkylaminogruppen ausgewählt sind,
deren Stereoisomere, N-Oxide und Additionssalze mit einer pharmazeutisch annehmbaren Säure oder Base.

2. Verbindungen nach Anspruch 1, worin X die Gruppe -N-R₄ darstellt und m den Wert 1 besitzt, deren Stereoisomere, N-Oxide und Additionssalze mit einer pharmazeutisch annehmbaren Säure oder Base.

3. Verbindungen nach Anspruch 1, worin X Sauerstoff bedeutet und m den Wert 1 besitzt, deren Stereoisomere. N-Oxide und Additionssalze mit einer pharmazeutisch annehmbaren Säure oder Base.

4. Verbindung nach einem der Ansprüche 1 bis 2, nämlich 1-(2,4-Dichlorbenzyl)-2-(4,5-dihydro[1H]imidazol-2-yl)-4-methylpiperazin, dessen Stereoisomere, N-Oxide und Additionssalze mit einer pharmazeutisch annehmbaren Säure.

5. Verbindung nach einem der Ansprüche 1 bis 2, nämlich 1-Methyl-2-(4,5-dihydro[1H]imidazol-2-yl)-4-(2-chlorbenzyl)-piperazin, dessen Stereoisomere, N-Oxide und Additionssalze mit einer pharmazeutisch annehmbaren Säure.

6. Verbindung nach einem der Ansprüche 1 bis 2, nämlich 1-Methyl-2-(4,5-dihydro[1H]imidazol-2-yl)-4-(2,4-dichlorbenzyl)-piperazin, dessen Stereoisomere, N-Oxide und Additionssalze mit einer pharmazeutisch annehmbaren Säure.

7. Verbindung nach einem der Ansprüche 1 bis 2, nämlich 1,4-Dibenzyl-2-[(4,5-dihydro[1H]imidazol-2-yl)-methyl]-piperazin, dessen Stereoisomere, N-Oxide und Additionssalze mit einer pharmazeutisch annehmbaren Säure.

8. Verbindung nach einem der Ansprüche 1 und 3, nämlich 1-methyl-4-(2-chlorbenzyl)-2-(4,5-dihydro[1H]oxazol-2-yl)-piperazin, dessen Stereoisomere, N-Oxide und Additionssalze mit einer pharmazeutisch annehmbaren Säure.

9. Verbindung nach einem der Ansprüche 1 und 3, nämlich 1,4-Dibenzyl-2-[(4,5-dihydro[1H]oxazol-2-yl)-methyl]-piperazin. dessen Stereoisomere, N-Oxide und Additionssalze mit einer pharmazeutisch annehmbaren Säure.

10. Verbindung nach einem der Ansprüche 1 bis 2, nämlich 1,4-Diisopropyl-2-(4,5-dihydro[1H]imidazol-2-yl)-piperazin, dessen Stereoisomere, N-Oxide und Additionssalze mit einer pharmazeutisch annehmbaren Säure.

11. Verfahren zur Herstellung der Verbindungen der Formel (I) nach Anspruch 1, **dadurch gekennzeichnet,** daß man:
a) - entweder die Verbindung der Formel (II):
C₆H₅-CH₂-NH-CH₂-CH₂-NH-CH₂-C₆H₅ (II)
in einem aromatischen Lösungsmittel in Gegenwart von Triethylamin mit dem Dibromid der Formel (III): 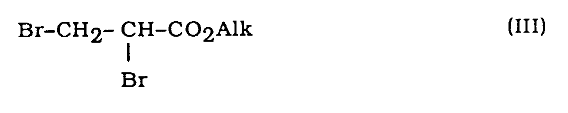 in der Alk eine geradkettige oder verzweigte Alkylgruppe mit 1 bis 4 Kohlenstoffatomen bedeutet,
zum Sieden am Rückfluß erhitzt zur Bildung der Verbindung der Formel (IV): 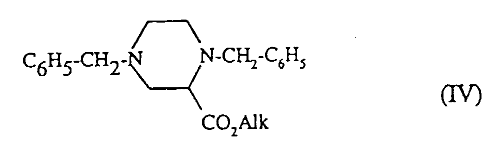 in der Alk die oben angegebenen Bedeutungen besitzt,
die anschließend durch Einwirkung von Wasserstoff in Gegenwart eines Katalysators debenzyliert wird zur Bildung der Verbindung der Formel (V): 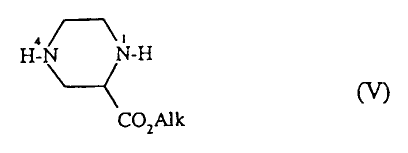 in der Alk die oben angegebenen Bedeutungen besitzt,
welche anschließend in die Verbindung der Formel (Va), (Vb) oder (Vc) umgewandelt wird: 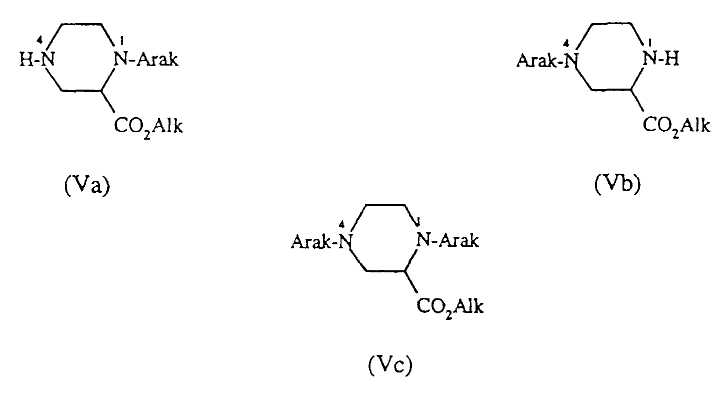 in denen Arak eine gegebenenfalls substituierte Arylalkylgruppe, wie sie oben definiert worden ist, bedeutet und Alk die oben angegebenen Bedeutungen besitzt,
- für die Verbindung der Formel (Va):
nach dem Schutz des Stickstoffatoms in der 4-Stellung mit Triphenylmethylchlorid in einem geeigneten Lösungsmittel in Gegenwart von Triethylamin durch Behandeln mit Arak-Cl in Gegenwart von Kaliumcarbonat und einer katalytischen Menge Kaliumiodid in einem geeigneten Lösungsmittel und dann Abspalten der Schutzgruppe von dem Stickstoffatom in der 4-Stellung mit gasförmigem Chlorwasserstoff in einem geeigneten Lösungsmittel,
- für die Verbindungen der Formeln (Vb) und (Vc):
durch Behandeln der Verbindung der Formel (V) direkt mit Arak-Cl in Toluol am Rückfluß in Gegenwart von Triethylamin und anschließend Trennung der mono-und disubstituierten Derivate durch Säulenchromatographie über Kieselgel,
b) - oder die Verbindung der Formel (VI): 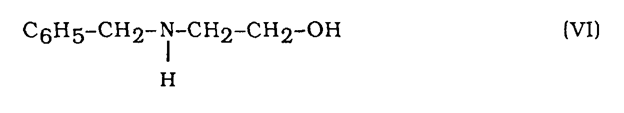 gegebenenfalls mit einem Keton oder einem Aldehyd, wobei das ausgewählte Keton und der ausgewählt Aldehyd homolog ist zu der Gruppe R'₁ (worin R'₁ die gleichen Bedeutungen besitzt wie R₁ mit Ausnahme von Wasserstoff), welche man auf das Stickstoffatom aufzupfropfen wünscht, in saurem und alkoholischem Medium behandelt, so daß man die Verbindung der Formel (VII) erhält: 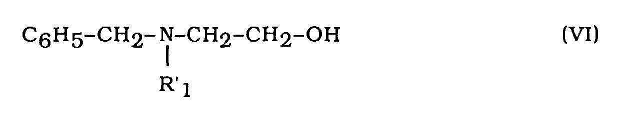 in der R'₁ die oben angegebenen Bedeutungen besitzt,
welche mit einem Chlorierungsmittel behandelten Verbindungen der Formel (VI) und (VII) die entsprechenden Chlorderivate ergeben und unter der Einwirkung einer wäßrigen gesättigten Ammoniaklösung zu der Verbindung der Formel (VIII) führen: 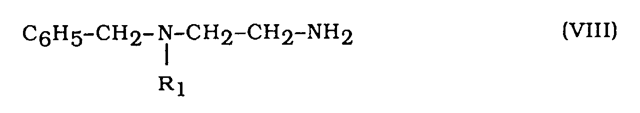 in der R₁ die oben angegebenen Bedeutungen besitzt,
die anschließend gegebenenfalls, wie die Verbindung der Formel (VI), mit einem Homologen der Gruppe R'₂ (worin R'₂ die gleichen Bedeutungen besitzt wie R₂ mit Ausnahme von Wasserstoff) in Form des Aldehyds oder des Ketons behandelt wird und nach dem Hydrieren der gebildeten Doppelbindung das Diamin der Formel (IX) liefert: 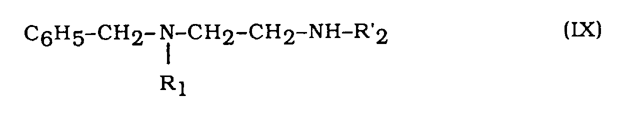 in der R₁ und R'₂ die oben angegebenen Bedeutungen besitzen,
welche Verbindungen der Formeln (VIII) und (IX) anschließend gemäß dem für die Verbindung der Formel (IV) beschriebenen Verfahren debenzyliert und dann mit dem oben beschriebenen Dibromid der Formel (III) behandelt werden, so daß man nach der eventuellen Trennung der Isomeren durch Chromatographie und/oder Kristallisation die Verbindung der Formel (X) erhält: 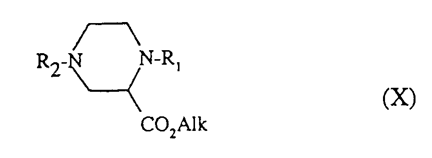 in der R₁, R₂ und Alk die oben angegebenen Bedeutungen besitzen,
c) - oder die Verbindung der Formel (VI): 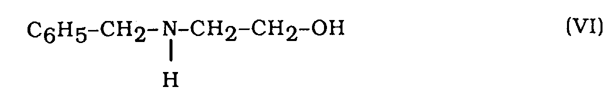 gegebenenfalls mit einem Keton oder einem Aldehyd, wobei das ausgewählte Keton oder der ausgewählte Aldehyd das Homologe der Gruppe R'₂ (worin R'₂ die gleichen Bedeutungen besitzt wie R₂ mit Ausnahme von Wasserstoff), welches man auf das Stickstoffatom aufzupfropfen wünscht, ist, in saurem alkoholischem Medium behandelt zur Bildung der Verbindung der Formel (XI): 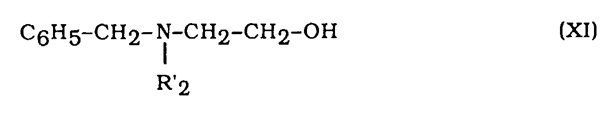 in der R'₂ die oben angegebenen Bedeutungen besitzt,
welche mit einem Chlorierungsmittel behandelten Verbindungen der Formeln (VI) und (XI) die entsprechenden Chlorderivate ergeben, welche nach der Behandlung mit Benzylamin zu dem Diamin der Formel (XII) führen: 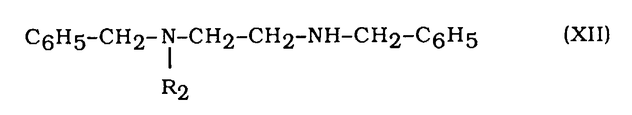 in der R₂ die oben angegebenen Bedeutungen besitzt,
welches durch Reaktion mit einem Halogenderivat der Formel (XIII): 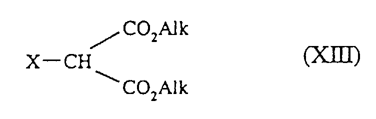 in der X ein Halogenatom darstellt und Alk die oben angegebenen Bedeutungen besitzt,
zu dem Diamin der Formel (XIV) führt: 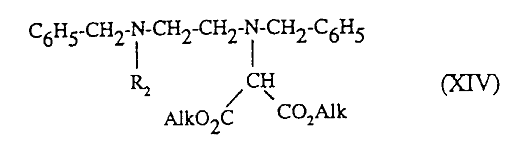 in der Alk und R₂ die oben angegebenen Bedeutungen besitzen,
welches nach der Metallisierung und Reaktion mit einem Halogenderivat der Formel (XV):
X - R₃ (XV)
in der X und R₃ die oben angegebenen Bedeutungen besitzen,
zu dem Diamin der Formel (XVI) führt: 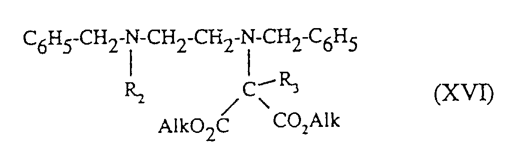 in der R₂, R₃ und Alk die oben angegebenen Bedeutungen besitzen,
welches nach der katalytischen Debenzylierung und der Cyclisierung zu dem Piperazinon der Formel (XVII) führt: 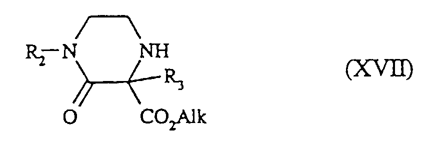 in der R₂, R₃ und Alk die oben angegebenen Bedeutungen besitzen,
welches:
- entweder durch Einwirkung eines Aldehyds oder eines Ketons, welche Vorläufer der Gruppe R'₁ darstellen, gemäß der oben beschriebenen Methode,
- oder durch direkte Alkylierung mit einem Halogenderivat der Formel (XVIII):
R'₁ - X (XVIII)
in der R'₁ und X die oben angegebenen Bedeutungen besitzen,
zu dem Piperazinon der allgemeinen Formel (XIX) führt: 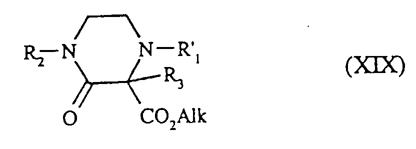 in der R'₁, R₂, R₃ und Alk die oben angegebenen Bedeutungen besitzen,
welche Piperazinone der Formeln (XIX) und (XVII) durch Reduktion mit Borhydriden zu dem Piperazin der Formel (XX) führen: 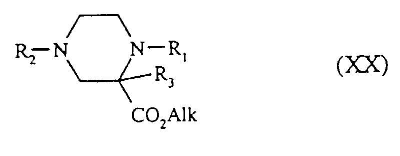 in der R₁, R₂, R₃ und Alk die oben angegebenen Bedeutungen besitzen,
wobei die sekundären Amine der Verbindungen der Formeln (V), (Va) und (Vb) gegebenenfalls:
- entweder durch Einwirkung von Aldehyden oder Ketonen, welche Vorläufer der Reste R'₁ oder R'₂ darstellen, gemäß der oben beschriebenen Methode,
- oder durch direkte Alkylierung mit einem Halogenderivat der Formel (XXI):
R - X (XXI)
in der R die gleiche Bedeutung besitzt wie R'₁ oder R'₂ und X ein Halogenatom darstellt,
substituiert werden können,
wobei im Fall der Verbindungen der Formel (V) das reaktivste Stickstoffatom gegebenenfalls zuvor mit einer Triphenylmethylgruppe geschützt werden kann, wie es für die Bildung der Verbindung der Formel (Va) beschrieben worden ist,
wobei die Verbindungen der Formeln (V), (Va), (Vb), (Vc), deren eventuelle Substitutionsprodukte, wie sie oben angesprochen worden sind, sowie die Verbindungen der Formel (X) ebenfalls Teil der Verbindungen der Formel (XX) darstellen, welche Verbindungen der Formel (XX) gegebenenfalls in ihre höheren Homologen der Formel (XXII) umgewandelt werden können: 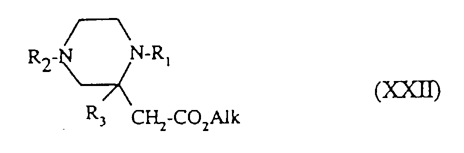 in der R₁, R₂, R₃ und Alk die oben angegebenen Bedeutungen besitzen,
durch Behandeln mit einem Reduktionsmittel zur Umwandlung der Esterfunktion in die Alkoholfunktion, durch Chlorierung, gefolgt von einer Behandlung mit einem Erdalkalimetallcyanid und schließlich durch Umwandlung des in dieser Weise erhaltenen Nitrils durch Einwirkung eines Alkohols in saurem Medium in einen Ester,
welche Verbindung der Formel (XXII), die gegebenenfalls der gleichen Behandlung unterworfen wird, zu einem Homologen der Formel (XXIII) führt: 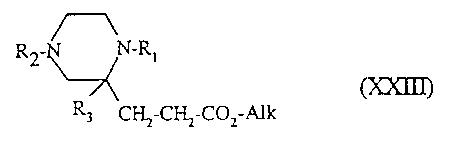 in der R₁, R₂, R₃ und Alk die oben angegebenen Bedeutungen besitzen,
wobei die Gesamtheit der Verbindungen der Formeln (XX), (XXII) und (XXIII) gemeinsam die Verbindungen der Formel (XXIV) bilden: 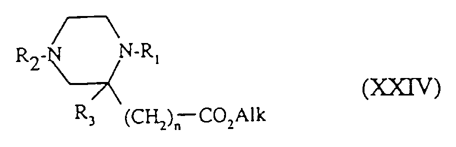 in der R₁, R₂, R₃, Alk und n die oben angegebenen Bedeutungen besitzen, welche:
**entweder**: a) der Einwirkung eines Diamins der Formel (XXV): 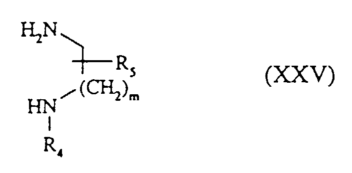 in der R₄, R₅ und m die oben angegebenen Bedeutungen besitzen,
in Gegenwart eines Trialkylaluminiums in einem wasserfreien aprotischen Lösungsmittel unterworfen werden zur Bildung der Verbindung der Formel (Ia): 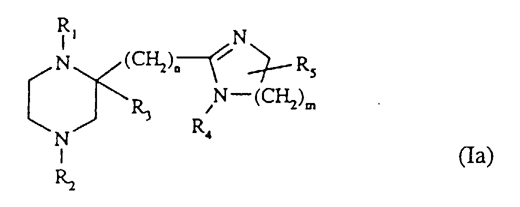 in der R₁, R₂, R₃, R₄, R₅, n und m die oben angegebenen Bedeutungen besitzen,
**oder**: b) der Einwirkung der Verbindung der Formel (XXVI): 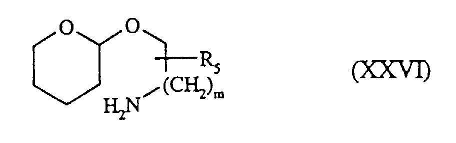 in der R₅ und m die oben angegebenen Bedeutungen besitzen,
unterworfen werden,
einer Verbindung, die man ausgehend von dem entsprechenden α-ω-Hydroxyamin erhält, dessen Aminogruppe zuvor durch Einwirkung von Benzylchlorid in das N,N-Dibenzyl-α-ω-hydroxyamin geschützt worden ist, und Schutz der Alkoholfunktion durch Dihydropyran und anschließende Hydrogenolyse in Gegenwart von Palladium-auf-Kohlenstoff zur Freisetzung der Amingruppe, welche Verbindung der Formel (XXVI) unter den gleichen Bedingungen wie die Verbindung der Formel (XXV) mit der Verbindung der Formel (XXIV) reagiert zur Bildung der Verbindung der Formel (XXVII): 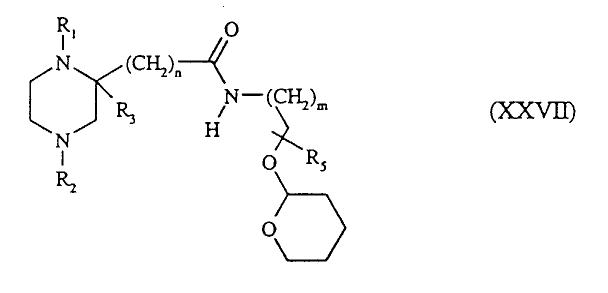 in der R₁, R₂, R₃, R₅, n und m die oben angegebenen Bedeutungen besitzen,
eine Verbindung, deren Alkoholfunktion in alkoholischem saurem Medium freigesetzt und dann unter der Einwirkung von Thionylchlorid in das entsprechende Chlorid umgewandelt wird, um die angestrebte Cyclisierung in basischem Medium zu ermöglichen und die Verbindung der Formel (Ib) zu erhalten: 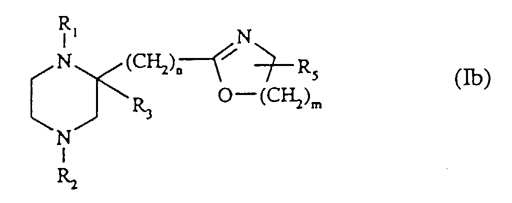 in der R₁, R₂, R₃, R₅, n und m die oben angegebenen Bedeutungen besitzen,
wobei die Gesamtheit der Verbindungen der Formeln (Ia) und (Ib) gemeinsam die Verbindungen der Formel (I) bilden, welche gereinigt und gegebenenfalls mit Hilfe einer klassischen Trennungstechnik in ihre Stereoisomeren aufgetrennt werden und die gewünschtenfalls in ihre N-Oxide oder ihre Additionssalze mit einer pharmazeutisch annehmbaren Säure oder Base umgewandelt werden.

12. Pharmazeutische Zubereitungen, enthaltend als Wirkstoff mindestens eine Verbindung nach einem der Ansprüche 1 bis 10 allein oder in Kombination mit einem oder mehreren inerten, nichttoxischen, pharmazeutisch annehmbaren Trägermaterialien.

13. Pharmazeutische Zubereitungen nach Anspruch 12, welche die Glucoseverträglichkeit von nicht-insulinabhängigen Diabetikern erhöhen und bei der Behandlung dieses Diabetes nützlich sind.

## Claims

1. Compounds of formula (I): 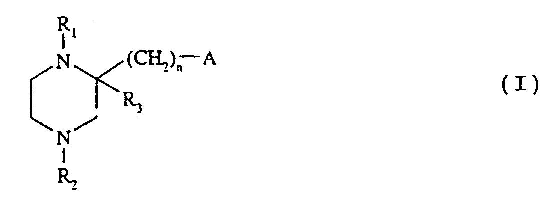 in which :
- R₁ and R₂, each independently of the other, are selected from hydrogen and alkyl, cycloalkyl, monocycloalkylalkyl, dicycloalkylalkyl, aryl and arylalkyl radicals, each of those radicals being optionally substituted,
- R₃ is selected from hydrogen and an alkyl radical,
- n has a value selected from 0, 1 and 2, and
- A represents the radical 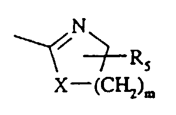 in which
- X represents oxygen or the group N-R₄,
- m has a value selected from 1 and 2,
- R₄ is selected from hydrogen and optionally substituted alkyl, alkoxycarbonyl and aryloxycarbonyl radicals, and
- R₅ is selected from hydrogen and an alkyl radical,
wherein:
- the terms "alkyl", "alkoxy", "monocycloalkylalkyl", "dicycloalkylalkyl", "arylalkyl" and "alkoxycarbonyl" denote radicals the hydrocarbon chain of which contains from 1 to 10 carbon atoms in a straight or branched chain,
- the term "cycloalkyl" denotes cyclic hydrocarbon radicals containing from 3 to 8 carbon atoms,
- the terms "aryl", "arylalkyl" and "aryloxycarbonyl" relate to radicals the aromatic part of which is a phenyl radical or a naphthyl radical, and
- the expression "optionally substituted" means that the radicals so described may be substituted by one or more chemical entities selected from alkyl, hydroxy, alkoxy, halogen, haloalkyl, polyhaloalkyl, nitro, amino, alkylamino and polyalkylamino radicals,
their stereoisomers, N-oxides and addition salts with a pharmaceutically acceptable acid or base.

2. Compounds according to claim 1 in which X represents the group -N-R₄ and m is 1, their stereoisomers, N-oxides and addition salts with a pharmaceutically acceptable acid or base.

3. Compounds according to claim 1 in which X represents oxygen and m is 1, their stereoisomers, N-oxides and addition salts with a pharmaceutically acceptable acid or base.

4. A compound according to either claim 1 or claim 2 which is 1-(2,4-dichlorobenzyl)-2-(4,5-dihydro[1H]-imidazol-2-yl)-4-methylpiperazine, its stereoisomers, N-oxides and addition salts with a pharmaceutically acceptable acid.

5. A compound according to either claim 1 or claim 2 which is 1-methyl-2-(4,5-dihydro[1H]imidazol-2-yl)-4-(2-chlorobenzyl)piperazine, its stereoisomers, N-oxides and addition salts with a pharmaceutically acceptable acid.

6. A compound according to either claim 1 or claim 2 which is 1-methyl-2-(4,5-dihydro[1H]imidazol-2-yl)-4-(2,4-dichlorobenzyl)piperazine, its stereoisomers, N-oxides and addition salts with a pharmaceutically acceptable acid.

7. A compound according to either claim 1 or claim 2 which is 1,4-dibenzyl-2-[(4,5-dihydro[1H]imidazol-2-yl)-methyl]piperazine, its stereoisomers, N-oxides and addition salts with a pharmaceutically acceptable acid.

8. A compound according to either claim 1 or claim 3 which is 1-methyl-4-(2-chlorobenzyl)-2-(4,5-dihydro[1H]-oxazol-2-yl)piperazine, its stereoisomers, N-oxides and addition salts with a pharmaceutically acceptable acid.

9. A compound according to either claim 1 or claim 3 which is 1,4-dibenzyl-2-[(4,5-dihydro[1H]oxazol-2-yl)-methyl]piperazine, its stereoisomers, N-oxides and addition salts with a pharmaceutically acceptable acid.

10. A compound according to either claim 1 or claim 2 which is 1,4-diisopropyl-2-(4,5-dihydro[1H]imidazol-2-yl)piperazine, its stereoisomers, N-oxides and addition salts with a pharmaceutically acceptable acid.

11. Process for the preparation of compounds of formula (I) according to claim 1, characterised in that:
a) - the compound of formula (II):
C₆H₅-CH₂-NH-CH₂-CH₂-NH-CH₂-C₆H₅ (II)
is brought to reflux in an aromatic solvent in the presence of triethylamine with the dibromide of formula (III): 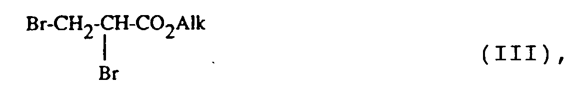 in which Alk represents a linear or branched alkyl radical having from 1 to 4 carbon atoms,
to give the compound of formula (IV): 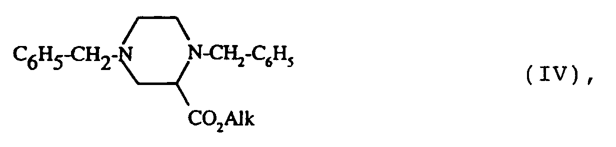 in which Alk is as defined above,
which is then debenzylated by the action of hydrogen in the presence of a catalyst to give the compound of formula (V): 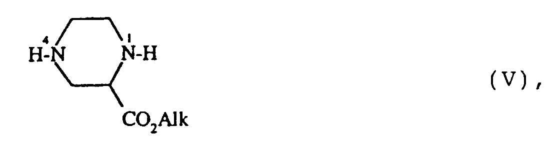 in which Alk is as defined above,
which is then converted into the compound of formula (Va), (Vb) or (Vc): 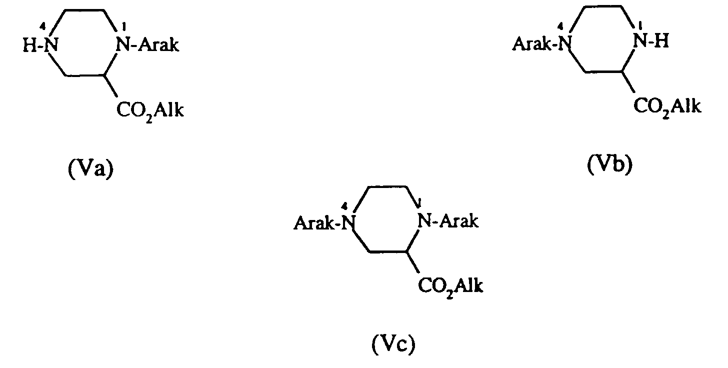 in which Arak represents an optionally substituted arylalkyl radical as defined above, and Alk is as defined above,
- for the compound of formula (Va):
after protection of the nitrogen atom in the 4-position by triphenylmethyl chloride in a suitable solvent, in the presence of triethylamine, by treatment with Arak-Cl in the presence of potassium carbonate and of a catalytic amount of potassium iodide, in a suitable solvent, and then deprotection of the nitrogen atom in the 4-position by means of gaseous hydrogen chloride in a suitable solvent,
- for the compounds of formulae (Vb) and (Vc):
by treatment of the compound of formula (V) directly with Arak-Cl in toluene at reflux in the presence of triethylamine, and then separation of the mono- and disubstituted compounds by chromatography over a silica gel column,
b) - or, the compound of formula (VI): 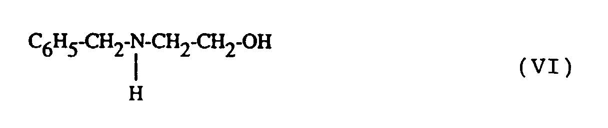 is optionally treated with a ketone or an aldehyde, in an acidic and alcoholic medium, the ketone or aldehyde chosen being the homologue of the radical R'₁ (R'₁ having the same definition as R₁, with the exception of hydrogen) that is to be bonded to the nitrogen atom, in order to obtain the compound of formula (VII): 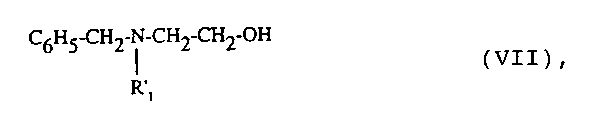 in which R'₁ is as defined above,
which compounds of formulae (VI) and (VII), when treated with a chlorinating agent, yield the corresponding chlorinated compounds and then, under the action of a saturated aqueous ammonia solution, yield the compound of formula (VIII): 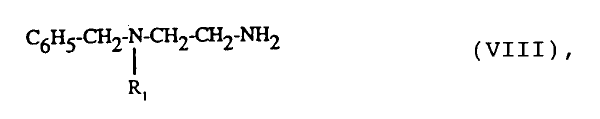 in which R₁ is as defined above,
which compound is then optionally treated, like the compound of formula (VI), with the homologue of the radical R'₂ (R'₂ having the same definition as R₂, with the exception of hydrogen) in the form of an aldehyde or ketone, and, after hydrogenation of the double bond that is formed, yields the diamine of formula (IX): 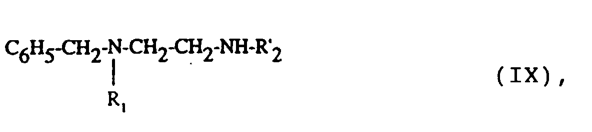 in which R₁ and R'₂ are as defined above,
the compounds of formulae (VIII) and (IX) then being debenzylated according to the process described for the compound of formula (IV) and then treated with the dibromide of formula (III) described above to give, after separation of any isomers by chromatography and/or crystallisation, the compound of formula (X): 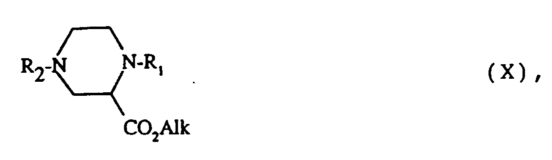 in which R₁, R₂ and Alk are as defined above,
c) - or, the compound of formula (VI): 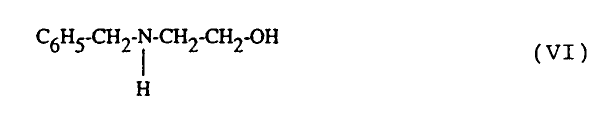 is optionally treated with a ketone or an aldehyde, in an acidic and alcoholic medium, the ketone or aldehyde chosen being the homologue of the radical R'₂ (R'₂ having the same definition as R₂, with the exception of hydrogen) that is to be bonded to the nitrogen atom, in order to obtain the compound of formula (XI): 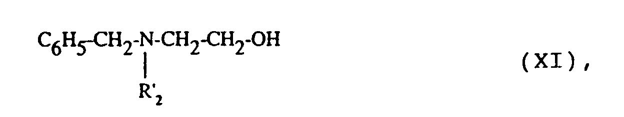 in which R'₂ is as defined above,
which compounds of formulae (VI) and (XI), when treated with a chlorinating agent, yield the corresponding chlorinated compounds which, when treated with benzylamine, yield the diamine of formula (XII): 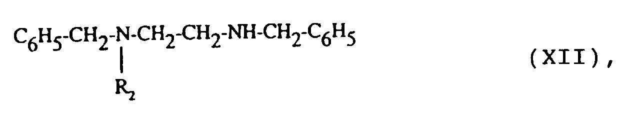 in which R₂ is as defined above,
which, by reaction with a halogenated compound of formula (XIII): 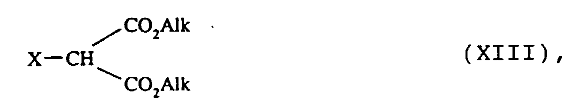 in which X represents a halogen atom and Alk is as defined above,
yields the diamine of formula (XIV): 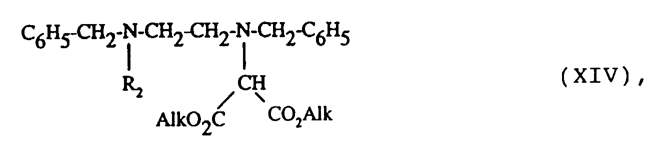 in which Alk and R₂ are as defined above,
which, after metallisation and reaction with a halogenated compound of formula (XV):
X-R₃ (XV),
in which X and R₃ are as defined above,
yields the diamine of formula (XVI): 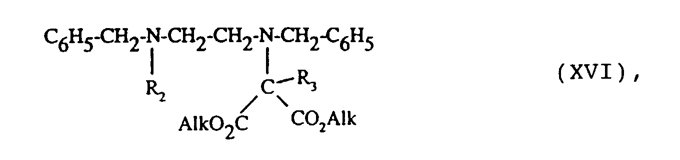 in which R₂, R₃ and Alk are as defined above,
which, after catalytic debenzylation and cyclisation, yields the piperazinone of formula (XVII): 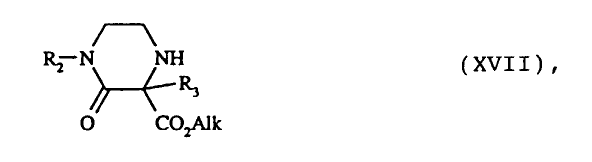 in which R₂, R₃ and Alk are as defined above,
which:
- either by the action of aldehyde or ketone precursors of the radical R'₁ according to the method described above,
- or by direct alkylation with a halogenated compound of formula (XVIII):
R'₁-X (XVIII),
in which R'₁ and X are as defined above,
yields the piperazinone of the general formula (XIX): 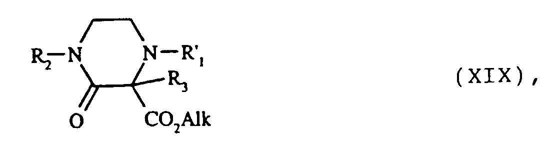 in which R'₁, R₂, R₃ and Alk are as defined above,
which piperazinones of formulae (XIX) and (XVII), by reduction with boron hydrides, yield the piperazine of formula (XX): 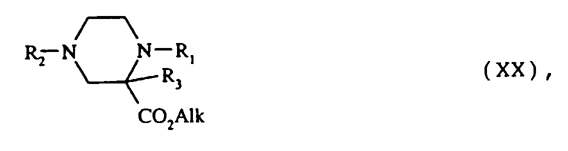 in which R₁, R₂, R₃ and Alk are as defined above,
it likewise being possible for the secondary amines of the compounds of formulae (V), (Va) and (Vb) to be substituted:
- either by the action of aldehyde or ketone precursors of the radicals R'₁ or R'₂ according to the method described above,
- or by direct alkylation with a halogenated compound of formula (XXI):
R-X (XXV),
in which R has the same definition as R'₁ or R'₂ and X represents a halogen,
with, in the case of the compounds of formula (V), optional protection beforehand of the most reactive nitrogen atom by a triphenylmethyl group, as indicated for the preparation of the compound of formula (Va),
the compounds of formulae (V), (Va), (Vb) and (Vc), their optional products of substitutions as discussed above, and the compounds of formula (X) likewise forming part of the compounds of formula (XX),
which compounds of formula (XX) may optionally be converted into their higher homologues of formula (XXII): 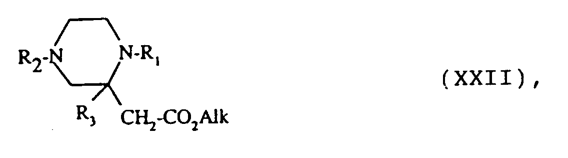 in which R₁, R₂, R₃ and Alk are as defined above,
by treatment with a reducing agent, in order to convert the ester function into an alcohol function, and then chlorination, followed by treatment with an alkaline earth metal cyanide, and finally conversion of the resulting nitrile into an ester by the action of an alcohol in an acidic medium,
which compound of formula (XXII), when optionally subjected to the same treatment, yields the homologue of formula (XXIII): 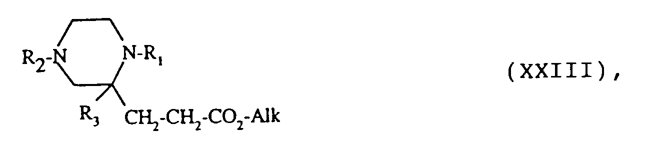 in which R₁, R₂, R₃ and Alk are as defined above,
all the compounds of formulae (XX), (XXII) and (XXIII) forming the compounds of formula (XXIV) in their entirety: 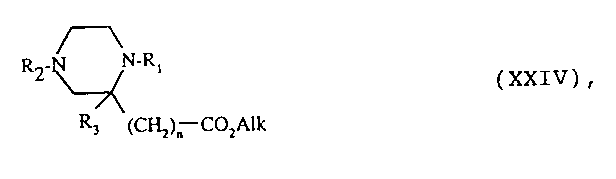 in which R₁, R₂, R₃, Alk and n are as defined above, which compounds of formula (XXIV) are subjected:
**either** : a) to the action of a diamine of formula (XXV): 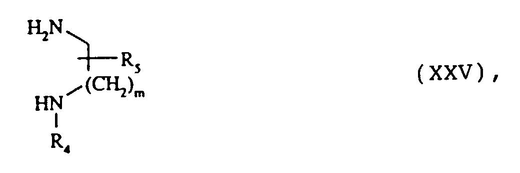 in which R₄, R₅ and m are as defined above,
in the presence of a trialkylaluminium, in an anhydrous aprotic solvent, to give the compound of formula (Ia): 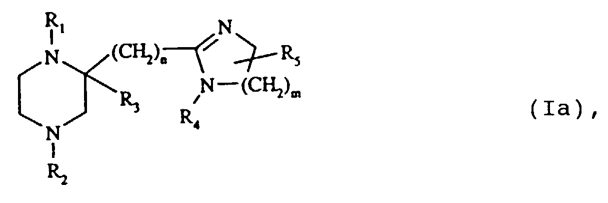 in which R₁, R₂, R₃, R₄, R₅, n and m are as defined above,
**or** : b) to the action of the compound of formula (XXVI): 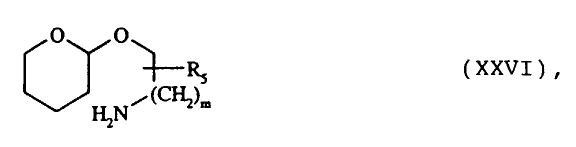 in which R₅ and m are as defined above,
which compound is obtained from the corresponding α-ω-hydroxy-amine, the amine function of which has previously been protected by conversion to N,N-dibenzyl-α-ω-hydroxy-amine, by the action of benzyl chloride, and then protection of the alcohol function by dihydropyran, and then hydrogenolysis in the presence of palladium-on-carbon in order to free the amine function,
the compound of formula (XXVI) reacting with the compound of formula (XXIV) under the same conditions as the compound of formula (XXV) to give the compound of formula (XXVII): 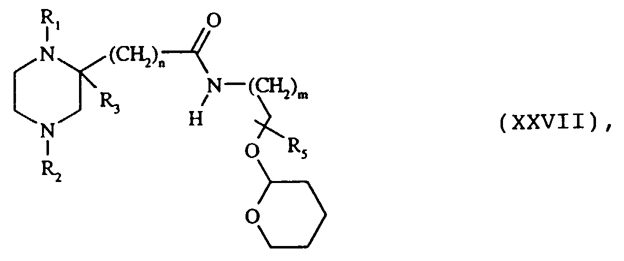 in which R₁, R₂, R₃, R₅, n and m are as defined above,
the alcohol function of which compound is freed in an acidic alcoholic medium and then converted into the corresponding chloride by the action of thionyl chloride in order to permit the desired cyclisation, in a basic medium, and obtain the compound of formula (Ib): 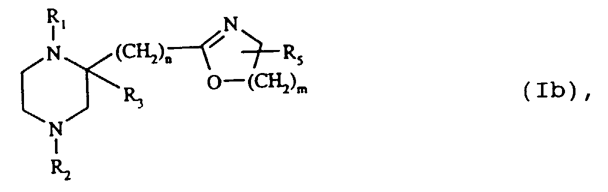 in which R₁, R₂, R₃, R₅, n and m are as defined above,
all the compounds of formulae (Ia) and (Ib) forming the compounds of formula (I) in their entirety, which compounds are purified and, optionally, separated into their stereoisomers by a conventional separation method and which are, if desired, converted into their N-oxides or into their addition salts with a pharmaceutically acceptable acid or base.

12. Pharmaceutical compositions containing as active ingredient at least one compound according to any one of claims 1 to 10, on its own or in combination with one or more inert, non-toxic, pharmaceutically acceptable carriers.

13. Pharmaceutical compositions according to claim 12 that increase glucose tolerance in patients suffering from non-insulin-dependent diabetes and that can be used in the treatment of the said diabetes.
